# FASCICULE DE BREVET EUROPEEN

(11) **EP 0 717 611 B1**
(45) Date de publication et mention de la délivrance du brevet: **12.08.1998**
(21) Numéro de dépôt: 94926283.6
(22) Date de dépôt: 07.09.1994
(51) Int. Cl.: A61J 1/20, A61M 5/46

(54) **DISPOSITIF DE SERINGUE POUR LE MELANGE DE DEUX COMPOSES**
SPRITZENVORRICHTUNG ZUM MISCHEN VON ZWEI PRÄPARATEN
SYRINGE DEVICE FOR MIXING TWO COMPOUNDS

(30) Priorité: 07.09.1993 FR 9310593; 15.10.1993 FR 9312271; 16.11.1993 FR 9313627; 24.12.1993 FR 9315639; 26.01.1994 FR 9400828; 11.03.1994 FR 9402844; 25.05.1994 FR 9406314
(43) Date de publication de la demande: 26.06.1996
(73) Titulaire: DEBIOTECH S.A., 1000 Lausanne 9 (CH)
(72) Inventeur: NEFTEL, Frédéric, CH-1000 Lausanne 9 (CH); BOUVIER, Bernard, F-95610 Eragny-sur-Oise (FR)
(74) Mandataire: Dronne, Guy
(86) Numéro de dépôt international: FR9401053
(87) Numéro de publication internationale: WO9507066

(56) Documents cités:
- EP-A- 0 022 977
- WO-A-93/02723
- DE-A- 1 913 926
- GB-A- 2 211 104
- US-A- 3 336 924
- US-A- 4 031 895
- US-A- 4 303 071
- US-A- 4 516 967
- US-A- 4 909 290
- US-A- 5 125 908
- US-A- 5 247 972

## Description

La présente invention a pour objet un dispositif de seringue permettant le mélange de deux composés et l'injection du mélange ainsi obtenu.

De façon plus précise, l'invention concerne un dispositif qui permet le stockage sous forme séparée de deux composés qui devront être mélangés avant leur injection.

On sait qu'il existe, notamment dans le domaine médical, des produits qui doivent être stockés séparément et qui ne doivent être mélangés qu'au moment de leur injection à un patient par exemple par voie sous-cutanée, intraveineuse ou intramusculaire. C'est en particulier le cas de produits médicamenteux qui sont préparés sous forme lyophilisée ou sous forme de poudre, et qui doivent être mélangés à un solvant, par exemple du sérum physiologique pour permettre leur administration au patient.

Dans d'autres cas, les deux composés à mélanger peuvent être des liquides, ces liquides ne pouvant être conservés longtemps sous forme mélangée.

La solution la plus fréquemment utilisée consiste à stocker la poudre dans un récipient séparé qui est obturé par une membrane étanche perforable à l'aide d'une aiguille. Par ailleurs, à l'aide d'une seringue, on vient prélever dans un deuxième récipient une certaine quantité de solvant que l'on injecte dans le premier récipient à travers la membrane. On mélange alors l'ensemble dans le premier récipient et à l'aide de la même seringue on aspire le mélange. La seringue est alors prête pour procéder à l'injection du médicament moyennant, dans la plupart des cas, un changement d'aiguille.

La solution décrite ci-dessus présente de nombreux inconvénients : d'une part, elle nécessite l'emploi du récipient obturé par la membrane perforable, de la seringue et du deuxième récipient contenant le solvant qui sont séparés. En outre, les manipulations requises sont relativement complexes. D'autre part, les conditions d'asepsie ne sont pas toujours aisées à respecter lors des différentes manipulations des deux composants.

Le document US-A-4.031.895 décrit un dispositif de seringue pour mélanger deux produits comprenant une seringue, une pièce de guidage de la seringue et un flacon contenant un des produits.

La seringue présente une aiguille qui est excentrée par rapport au corps de seringue.

Le document US-A-4.516.967 décrit un dispositif de seringue comprenant également une seringue, une pièce de guidage et un flacon contenant un des produits à mélanger. L'étanchéité entre la seringue et la pièce de guidage est assurée par un ruban adhésif qui doit être enlevé pour permettre le déplacement de la seringue par rapport à la pièce de guidage.

Il faut également prendre en considération le fait que les installations de lyophilisation acceptent de préférence des récipients de forme standard en verre et à fond externe plat. Il est donc intéressant que la partie du dispositif de seringue destiné à recevoir le produit à lyophiliser présente cette forme. D'autre part, la plupart des médicaments actuels se présentent dores et déjà sous cette forme et toute modification de conditionnement nécessiterait une nouvelle autorisation de mise sur le marché avec des études de stabilité longues et coûteuses.

En outre, il est fondamental d'assurer un confinement de l'aiguille pendant toute la période de stockage et pendant les manipulations correspondant au mélange des produits.

L'objet de la présente invention est de fournir un dispositif de seringue selon le preambule de la revendication 1 qui permet les manipulations de mélange des deux produits en maintenant l'aiguille dans des conditions d'aseptie rigoureuses.

Pour atteindre ce but, le dispositif de seringue se caractérise en ce que lesdits moyens d'étanchéité incluent un joint monté à l'intérieur desdits moyens de guidage, ledit joint étant apte à assurer une étanchéité entre le corps de la seringue et la face interne des moyens de guidage en position de stockage et lors des déplacements relatifs des deux pièces.

De préférence, ledit flacon fait partie intégrante du dispositif de seringue. Cependant, on ne sortirait pas de l'invention si la seringue avec sa pièce de guidage était fournie indépendamment du flacon, les deux ensembles étant solidarisés ultérieurement.

Les premiers moyens d'étanchéité assurent une étanchéité dynamique entre les moyens de guidage et le corps de seringue durant tous les déplacements relatifs de ces deux pièces.

De préférence, ladite première extrémité des moyens de guidage comprend des moyens de solidarisation sur l'ouverture dudit flacon.

On comprend que, grâce aux dispositions de l'invention, on a un seul ensemble qui comporte, d'une part, le flacon contenant le produit, de préférence lyophilisé, et, d'autre part, la seringue qui contient le solvant. Cependant, les deux sont reliés entre eux par des moyens de guidage qui rendent aisée l'opération de mélange. En outre, on comprend que le flacon est un flacon de type standard utilisé lors des opérations classiques de lyophilisation puisque les moyens de guidage peuvent n'être mis en place qu'après la lyophilisation. En outre, grâce aux moyens d'étanchéité l'aiguille est protégée durant le stockage et lors des manipulations contre les risques de contamination.

Selon un premier mode de mise en oeuvre, les moyens de mise en communication comprennent une aiguille montée à l'extrémité d'injection de la seringue et dont la pointe est apte à perforer la zone centrale du bouchon lorsque l'extrémité de la seringue est amenée dans sa deuxième position.

On comprend ainsi que l'aiguille joue le double rôle de permettre la mise en communication entre l'intérieur du corps de la seringue et le flacon et celui d'aiguille classique d'injection une fois la seringue séparée du flacon.

De préférence également, le dispositif de seringue comporte en outre un protège-aiguille qui entoure l'aiguille sur toute sa longueur et qui présente une extrémité perforante s'étendant au-delà de la pointe de l'aiguille et qui est amovible par rapport à ladite aiguille, par quoi la perforation du bouchon du flacon est réalisée par la première extrémité du protège-aiguille.

Selon un deuxième mode de mise en oeuvre de l'invention, les moyens de mise en communication comprennent une pièce tubulaire disposée entre la zone centrale de bouchon et l'extrémité d'injection de la seringue, ladite pièce tubulaire présentant une première extrémité apte à obturer la zone centrale du bouchon et une deuxième extrémité apte à percer une cloison perforable ménagée à l'extrémité d'injection de la seringue lorsque celle-ci est amenée dans sa deuxième position.

On comprend que, dans ce deuxième mode de mise en oeuvre, la pièce tubulaire comporte deux extrémités perforantes qui perforent le bouchon du flacon et la cloison perforable de la seringue lors de l'enfoncement de la seringue dans les moyens de guidage permettant ainsi le mélange des deux composés, la cloison perforable est de préférence elle-même un bouchon perforable venant obturer ladite seringue.

Il en est de même lorsque, à l'intérieur de la seringue, on dispose une carpule équipée d'un bouchon perforable.

De préférence également, les moyens de guidage comportent en outre des deuxièmes moyens d'étanchéité aptes à assurer avec les premiers moyens d'étanchéité un confinement des moyens de mise en communication à l'intérieur desdits moyens de guidage.

De préférence également, les moyens de guidage comportent entre leur première et deuxième extrémités un orifice obturé par un filtre micro poreux aseptique.

La présence de ce filtre présente un double avantage. D'une part, il permet à une partie de l'air contenu dans les moyens de guidage de sortir ce qui facilite grandement la remise en place de la seringue dans les moyens de guidage tout en maintenant durant le stockage l'intérieur des moyens de guidage dans des conditions d'asepsie rigoureuses. D'autre part, il permet de procéder, à travers ce filtre, à l'introduction d'un gaz aseptisant après la fixation des moyens de guidage sur le flacon.

De préférence encore, le dispositif de seringue comprend un dispositif auto-injecteur qui est monté sur l'ensemble seringue/pièce de guidage après que cet ensemble ait été désolidarisé du flacon. Le dispositif auto-injecteur comprend un support, des moyens d'immobilisation en translation de ladite pièce de guidage, des premiers moyens poussoirs pour provoquer l'enfoncement de la seringue dans ladite pièce de guidage d'une première distance prédéterminée et des deuxièmes moyens poussoirs pour provoquer l'enfoncement du piston de la seringue dans le corps de la seringue d'une deuxième distance prédéterminée tout en ne modifiant pas la position du corps de la seringue, ladite première extrémité de la pièce de guidage formant une surface d'appui sur le corps du patient.

On comprend que l'action du premier poussoir permet d'enfoncer l'aiguille dans le corps du patient d'une profondeur prédéterminée alors que l'action du deuxième poussoir permet de procéder à l'injection du mélange.

De préférence encore, le dispositif de seringue comprend en outre des moyens annulaires de protection entourant une partie du corps de ladite seringue pour interdire l'accès manuel à la face externe de ladite partie du corps de seringue, une première extrémité desdits moyens de protection étant adjacente auxdits premiers moyens d'étanchéité lorsque ladite seringue occupe sa première position dans lesdits moyens de guidage, la longueur axiale l des moyens de protection entre sa première et sa deuxième extrémité étant au moins égale à la course l' de ladite seringue dans lesdits moyens de guidage entre sa première et sa deuxième position.

Selon un autre mode de réalisation, le joint d'étanchéité comprend des moyens temporaires de solidarisation en translation avec le corps de ladite seringue lorsque ladite seringue est déplacée entre sa première et sa deuxième position. Dans ce cas, les lèvres d'étanchéité sont extérieures et coopérent avec la pièce de guidage.

Selon encore un autre mode de réalisation, les premiers moyens d'étanchéité font partie intégrante du corps de seringue. De préférence encore, le corps de seringue est constitué par une carpule à l'extrémité de laquelle est fixé un embout en matériau plastique portant l'aiguille de la seringue. L'embout présente sur sa face externe au moins une nervure annulaire coopérant avec la face interne des moyens de guidage pour réaliser une étanchéité dynamique.

Selon encore un autre aspect de l'invention, de préférence, les moyens de guidage sont munis d'une chambre de confinement des gaz susceptibles de sortir du flacon lorsque l'aiguille est extraite de celui-ci.

D'autres caractéristiques et avantages de la présente invention apparaîtront mieux à la lecture de la description qui suit de plusieurs modes de réalisation de l'invention donnés à titre d'exemples non limitatifs. La description se réfère aux figures annexées sur lesquelles :
- la figure 1 est une vue en coupe longitudinale d'un premier mode de réalisation du dispositif de seringue dans sa position de stockage ;
- la figure 2 est une vue similaire à celle de la figure 1 montrant le dispositif de seringue dans une position permettant le mélange des composés ;
- la figure 3 est une vue en coupe longitudinale d'un deuxième mode de réalisation du dispositif de seringue, celui-ci étant dans sa position de stockage ;
- la figure 4 est une vue similaire à celle de la figure 3 montrant le dispositif de seringue dans la position de mélange des composés ;
- la figure 5 est une vue en coupe longitudinale d'un troisième mode de réalisation du dispositif de seringue ;
- la figure 6 est une vue partielle en coupe longitudinale d'un quatrième mode de réalisation du dispositif de seringue comportant un flacon de type modifié ;
- les figures 7a et 7b représentent une autre variante de réalisation de la pièce de guidage respectivement en coupe verticale et en section selon le plan BB de la figure 7a ;
- les figures 8a à 8c montrent encore une autre variante de réalisation du dispositif de seringue ;
- les figures 9a et 9b montrent deux variantes de réalisation d'une pièce de guidage munie d'une capsule de sertissage ;
- la figure 9c montre un dispositif de seringue comportant une pièce de guidage selon la figure 9b ;
- les figures 10a à 10d illustrent l'utilisation d'un dispositif auto-injecteur en liaison avec le dispositif de seringue ;
- les figures 11a à 11c sont analogues aux figures 8a à 8c mais montrent une variante de réalisation ;
- la figure 12 montre en coupe verticale un autre mode de réalisation du dispositif de seringue ;
- la figure 13 montre en coupe verticale un autre mode de réalisation du dispositif de seringue illustrant un autre mode de réalisation des moyens d'étanchéité entre le corps de seringue et la pièce de guidage ;
- la figure 14a montre une variante du mode de réalisation du dispositif de seringue selon la figure 12 ;
- les figures 14b et 14c sont des vues de détails de la figure 14a montrant le joint en position de stockage et d'utilisation ;
- la figure 15 illustre une première variante de réalisation de la chambre de confinement de gaz ;
- les figures 16a et 16b montrent deux autres variantes de réalisation de la chambre de confinement de gaz ;
- la figure 17 illustrent une variante de réalisation du dispositif de seringue permettant le confinement des gaz ;
- la figure 18a montre une partie du dispositif de seringue dans un mode de réalisation permettant de contrôler l'extraction de la seringue hors de la pièce de guidage ; et
- la figure 18b est une vue partielle en coupe verticale montrant le joint d'étanchéité de la figure 18a.

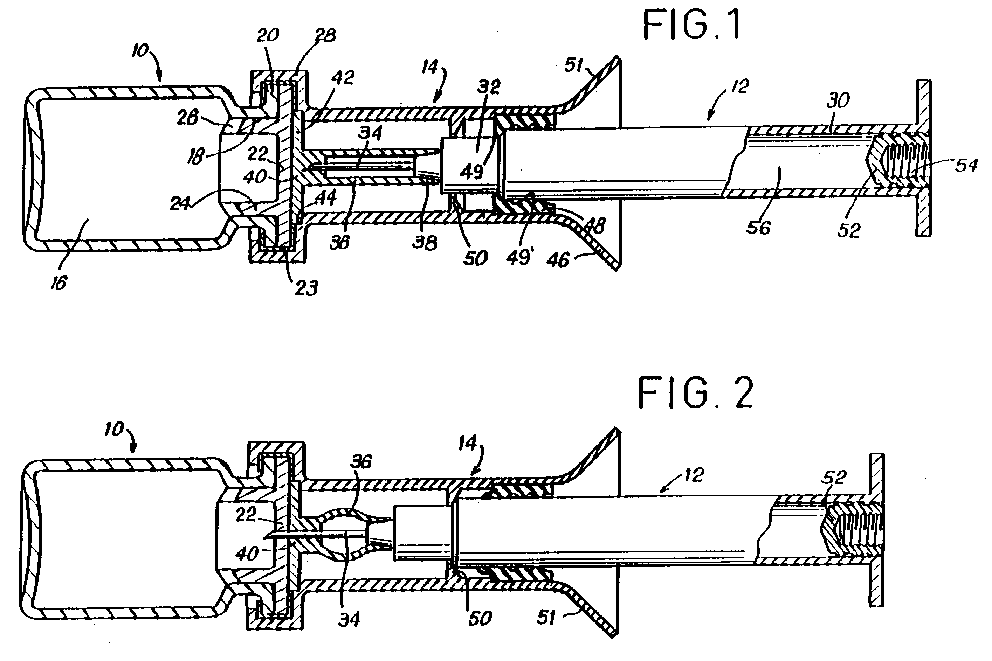

En se référant tout d'abord à la figure 1, on va décrire un premier mode de réalisation du dispositif de seringue. Celui-ci se compose essentiellement d'un flacon 10, d'une seringue 12 et d'un manchon de guidage 14 monté sur le flacon 10. Plus précisément, le flacon 10 qui est destiné à recevoir un premier composé à mélanger, référencé 16, qui est de préférence un composé lyophilisé, présente une ouverture 18 qui se termine de préférence par une collerette 20. Un bouchon 22 de type standard obture l'ouverture 18. Ce bouchon est réalisé en un matériau perforable et qui peut être rendu solidaire de façon étanche à la collerette 20 du flacon par l'intermédiaire d'une capsule en aluminium 23 qui laisse libre la partie centrale du bouchon, cette partie étant perforable.

De préférence, le bouchon 22 comporte un corps cylindrique 24 qui coopère avec l'ouverture 18 du flacon. Ce corps cylindrique 24 comporte des ouvertures axiales 26, telles que lorsque le bouchon est en partie engagé dans l'ouverture du flacon, les passages 24 permettent la circulation des gaz lors de l'opération de lyophilisation alors que, lorsque le bouchon est complètement enfoncé, il réalise une étanchéité parfaite.

La pièce de guidage 14 a, dans ce mode de réalisation, de préférence une forme cylindrique et comporte une première extrémité munie d'une bride de clipsage 28 sur le bouchon 22 et la collerette 20 du flacon assurant ainsi une solidarisation au moins en translation entre la pièce de guidage 14 et le flacon. La pièce de guidage a un diamètre légèrement supérieur à celui du corps 30 de la seringue 12. Comme cela est bien connu, le corps de la seringue 30 se termine par une extrémité d'injection 32 sur laquelle est montée une aiguille 34.

Dans le mode de réalisation représenté sur la figure 1, l'aiguille est protégée par un capuchon 36 dont l'extrémité 38 coopère de façon étanche avec l'extrémité d'injection 32 de la seringue et dont la deuxième extrémité 40 est perforable et se prolonge par une rondelle cylindrique 42 qui coopère avec une gorge annulaire 44 ménagée dans la bride 28 de fixation de la pièce de guidage 14. Ainsi l'extrémité 40 du capuchon de protection 36 est immobilisée en translation et plaquée contre la partie perforable 22 du bouchon du flacon 10. En outre, la rondelle 42 ayant sa périphérie pincée entre la périphérie du bouchon du flacon et la gorge 44 recouvre de façon étanche la partie perforable du bouchon et assure l'étanchéité de cette extrémité de la pièce de guidage 14. Comme on l'expliquera ultérieurement, d'autres modes de protection de l'aiguille peuvent être utilisés. L extrémité ouverte 46 de la pièce de guidage 14 est munie d'un joint d'étanchéité interne 48 qui est calibré de telle manière qu'il réalise une étanchéité entre la pièce de guidage 14 et le corps 30 de la seringue permettant un déplacement en translation de la seringue à l'intérieur de la pièce de guidage 14 tout en maintenant l'étanchéité pendant toute la translation. Pour réaliser cette étanchéité, le joint d'étanchéité élastique est de préférence en forme de bague et présente de préférence sur sa face interne trois lèvres d'étanchéité 49'. En outre, la longueur l de la bague selon la direction de déplacement de la seringue est suffisante pour assurer une solidarisation suffisante entre la seringue et la pièce de guidage 14 tout en autorisant le déplacement en translation de la seringue par rapport à la pièce de guidage sous l'effet d'une force appliquée à la seringue. La longueur l est par exemple égale à 1 cm. De plus, la longueur du joint aide au maintien en positions coaxiales de la pièce de guidage et du corps de la seringue lors du déplacement en translation de celle-ci. De préférence, le joint 48 défini un premier épaulement interne 49 escamotable qui limite l'enfoncement de la seringue dans la pièce de guidage lorsque le dispositif de seringue est stocké.

Comme le montre également la figure 1, de préférence, il est prévu à l'intérieur de la pièce de guidage 14 un deuxième épaulement 50 qui limite la possibilité d'enfoncement de la seringue 12 dans la pièce de guidage 14. L'épaulement 50 est défini de telle manière que, lorsque l'extrémité de la seringue arrive en butée sur celui-ci, l'extrémité de l'aiguille 34 a traversé la partie centrale 22 du bouchon mais ne fait que très légèrement saillie dans le flacon 10 afin de limiter au maximum le volume perdu dans le flacon lorsqu'on veut récupérer par aspiration, en position verticale, à l'aide de l'aiguille, le mélange réalisé dans le flacon. Enfin, comme cela est bien connu, la seringue 12 comporte un piston 52 monté coulissant dans le corps de la seringue. Ce piston 52 comporte un taraudage 54 dans lequel on peut venir fixer la tige de commande du piston 52. Dans sa position de stockage, le deuxième composé à mélanger est disposé à l'intérieur du volume 56 du corps de la seringue 12.

Selon une variante de réalisation, l'extrémité ouverte 46 de la pièce de guidage 14 se prolonge par une collerette tronconique évasée 51. Cette collerette a pour but de protéger les doigts de l'opérateur qui tient la pièce de guidage lors de la remise en place de la seringue dans cette pièce après injection au patient. Il va de soi que la collerette pourrait être remplacée par une autre forme faisant saillie hors de la face externe de la pièce de guidage et apte à protéger les doigts de l'opérateur placés du côté opposé à l'extrémité 46 dans laquelle est réintroduite la seringue après avoir été utilisée.

Selon une autre variante, la bride de clipsage 28 de la pièce de guidage fait toute la circonférence du bouchon 22 et assure par serrage la fixation étanche de la périphérie du bouchon 22 sur la collerette 20 du flacon. Cette disposition permet d'éviter la mise en place de la carpule métallique 23 représentée sur les figures 1 et 2.

Il est également possible de prévoir que le bouchon 22 et/ou le fond 40 du capuchon 36 de l'aiguille soient préperforés en leur centre. Cette caractéristique permet de réduire l'effort qui doit être appliqué au corps de la seringue pour que l'extrémité de l'aiguille traverse le capuchon et le bouchon. En outre, cette disposition permet de réduire la quantité de particules libérées du bouchon lorsque l'aiguille le traverse. Cela évite donc que ces particules ne soient injectées avec le mélange. En effet, le serrage du bouchon dans l'ouverture 18 du flacon assure une précontrainte du matériau constituant le bouchon. On obtient ainsi un bouchon 22 et/ou le fond du capuchon 36 qui, mis en place, est/sont étanche tout en présentant une préperforation.

De préférence également, la pièce de guidage 14 est réalisée en un matériau synthétique, par exemple du polypropylène, permettant sa stérilisation par des techniques classiques telles qu'autoclavage, radiations gamma, etc.

L'utilisation du premier mode de réalisation du dispositif de seringue représenté sur la figure 1 est la suivante : dans une première étape, on dispose le composé à lyophiliser dans le flacon 10 qui est alors dépourvu de la pièce de guidage 14. Le bouchon 22 est placé dans sa position semi-enfoncée. Lorsque l'opération de lyophilisation est terminée, on enfonce complètement le bouchon 22 et on met en place la capsule d'aluminium 24. On met indépendamment en place la seringue 12 contenant le deuxième composé liquide qui a été préalablement stérilisé avec son capuchon de protection 36 dans la pièce de guidage 14 puis cet ensemble est fixé sur le flacon par la coopération de la bride de la pièce de guidage 14 et de la collerette 20 du flacon 10. Le dispositif de seringue est alors dans son état représenté sur la figure 1 et est stocké dans cette position. En variante, on peut d'abord mettre en place la seringue dans la pièce de guidage. On remplit alors la seringue avec le liquide et on obture celle-ci avec le piston 52. Cet ensemble est stérilisé puis fixé sur le flacon 10. Eventuellement cet assemblage est réalisé sous hotte à flux laminaire.

Lorsqu'on veut procéder à l'injection du mélange du composé lyophilisé 16 avec le liquide 56 contenu dans la seringue 12, on opère de la manière suivante : on provoque l'enfoncement du corps de la seringue 12 dans la pièce de guidage 14 jusqu'à ce que le corps de la seringue arrive en butée sur l'épaulement 50. Dans cette position, le capuchon 36 qui est élastique s'est déformé et l'extrémité acérée de l'aiguille 34 à perforer le fond 40 du capuchon 36 et la partie perforable du bouchon 22 du flacon. C'est ce qui est représenté sur la figure 2. L'aiguille 36 permet ainsi la mise en communication de l'intérieur du corps de la seringue 12 et de l'intérieur du flacon 10. Il suffit alors de monter la tige de commande dans le piston 52 de la seringue et de provoquer l'enfoncement partiel de celui-ci pour faire passer une quantité convenable du liquide 56 à l'intérieur du flacon 10 pour produire la mélange des composés 16 et 56. Lorsque le mélange est effectivement obtenu dans le flacon 10, on provoque le retrait du piston 52 pour aspirer le mélange à l'intérieur du corps 30 de la seringue qui est placée verticalement. Celle-ci est alors prête à utilisation. Il suffit alors d'extraire la seringue 12 de la pièce de guidage 14. On comprend que lors de cette opération le capuchon 36 de l'aiguille 34 est maintenu sur le flacon 10 par la pièce de guidage 14. Il est possible de prévoir que l'aiguille qui a servi à perforer le bouchon et à réaliser le mélange soit détachable du corps de la seringue. Elle peut éventuellement alors être remplacée par une autre aiguille pour procéder à l'injection du mélange.

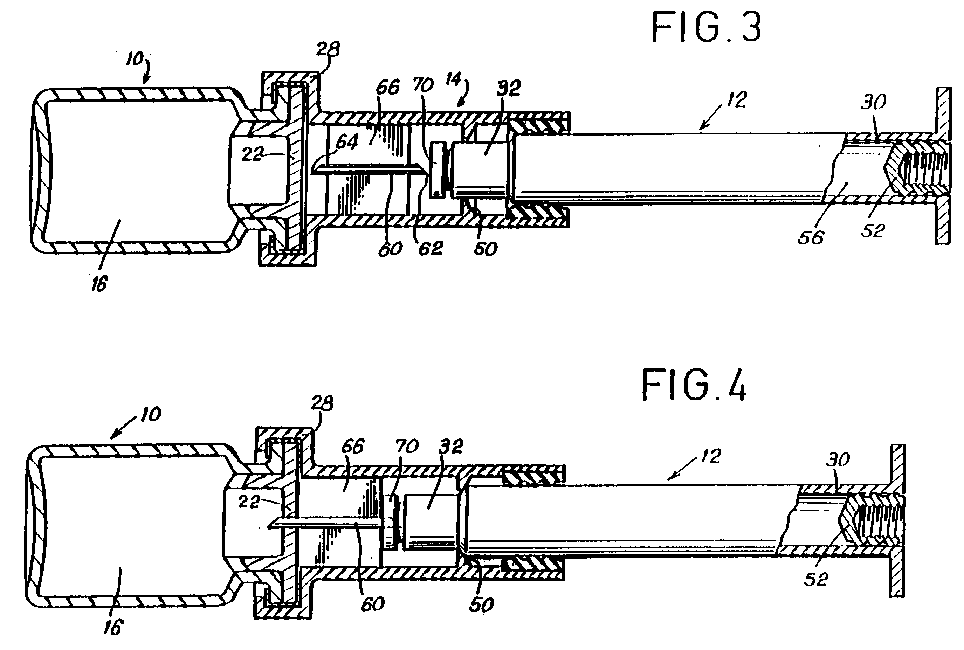

En se référant maintenant à la figure 3, on va décrire un deuxième mode de réalisation du dispositif de seringue. Dans ce deuxième mode, le flacon 10 destiné à recevoir le composé lyophilisé 16 et son bouchon 22 sont identiques à ceux de la figure 1. On ne les décrira pas à nouveau. Il en va de même pour la pièce de guidage 14 dont l'extrémité 28 est fixée sur le flacon 10. La différence consiste dans le fait que l'aiguille 34 est remplacée par une pièce tubulaire 60 qui présente deux extrémités perforantes 62 et 64, la pièce 60 étant montée à l'intérieur de la pièce de guidage 14 entre l'extrémité d'injection 32 de la seringue 12 et le bouchon 22 du flacon 10. Cette pièce tubulaire 60 est maintenue dans la position axiale de la pièce de guidage 14 par des ailettes rayonnantes 66 qui coopèrent avec la paroi interne de la pièce de guidage 14. En outre, l'extrémité d'injection 32 de la seringue est munie d'une cloison perforable 70 qui est donc disposée en regard de l'extrémité 62 de la pièce tubulaire 60.

En variante, la pièce tubulaire 60 pourrait être constituée par une double aiguille entre lesquelles on interpose un filtre de nature convenable. On peut également prévoir que, lors de l'extraction de la seringue hors de la pièce de guidage 14, l'ensemble des deux aiguilles soit maintenu en place à l'extrémité d'injection de la seringue.

En variante, il est possible d'interposer entre la partie de clipsage 28 de la pièce de guidage et la capsule 23 de fixation un joint d'étanchéité, par exemple un O' ring.

Pour utiliser le dispositif de seringue et procéder au mélange des composés contenus respectivement dans le corps de la seringue et dans le flacon 10, on provoque l'enfoncement du corps de la seringue dans la pièce de guidage 14 jusqu'à ce que le corps de seringue arrive en butée sur l'épaulement 50. Comme le montre mieux la figure 4, dans cette position, les extrémités acérées 62 et 64 de la pièce tubulaire 60 perforent respectivement la cloison perforable 70 de la seringue et la partie centrale du bouchon 22 du flacon. On voit également que, dans cette position, les faces d'extrémité des ailettes rayonnantes de guidage 66 servent de butée entre le bouchon 22 du flacon et la cloison perforable 70 de la seringue assurant effectivement la perforation de la cloison 70 et du bouchon 22. Dans cette position, il est possible de faire passer une partie du liquide contenu dans la seringue 12 en agissant sur son piston 52. Une fois que le mélange a été réalisé dans le flacon 10, il suffit d'exercer une traction sur le piston 52 pour aspirer le mélange dans le corps de la seringue placé verticalement. Ce même type de manipulation peut être réalisé avec une carpule préremplie du liquide ou avec une seringue équipée d'une carpule.

Pour procéder à l'injection, il suffit d'extraire la seringue 12 de la pièce de guidage 14 et de mettre en place une aiguille convenable à l'extrémité d'injection 32 de la seringue.

En variante, on peut prévoir que, pour procéder à l'injection du mélange, ce soit le flacon qui soit séparé de la pièce de guidage 14, cette dernière restant solidaire de l'extrémité de la seringue. Pour cela il suffit de prévoir pour la bride 28 de fixation de la pièce de guidage sur le flacon une découpe qui permette un déchirement de celle-ci, soit de façon manuelle soit par rotation de la pièce de guidage par rapport au flacon.

Dans ce cas, pour procéder à l'injection du mélange au patient par voie sous cutanée ou intramusculaire, il est nécessaire de procéder à un enfoncement supplémentaire de la seringue dans la pièce de guidage afin que la pointe de l'aiguille fasse effectivement saillie hors de la face inférieure de la bride de fixation, cette dernière étant au contact de la peau du patient. Pour contrôler cet enfoncement, il est intéressant de prévoir à l'intérieur de la pièce de guidage un épaulement supplémentaire, l'épaulement 50 étant escamotable sous l'effet de l'enfoncement de la seringue.

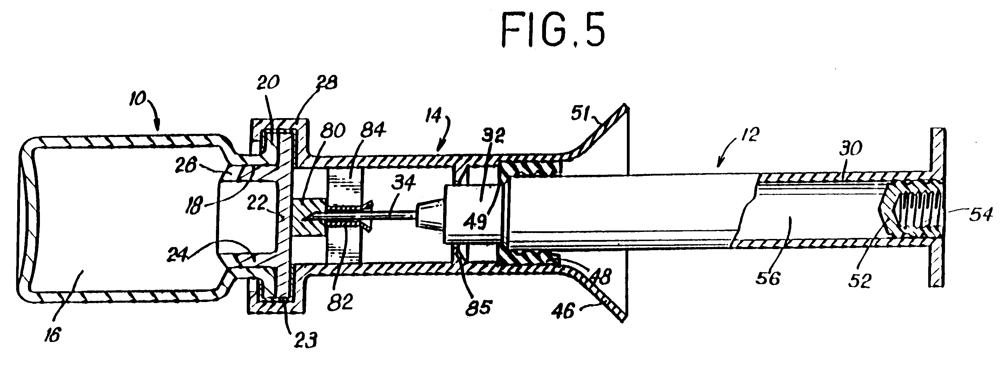

La figure 5 illustre un troisième mode de réalisation du dispositif de seringue. Selon ce mode de réalisation, le capuchon 36 de l'aiguille est remplacé par une pièce de préférence cylindrique perforable 80 qui est fixée dans l'extrémité de la pièce de guidage 14, la pointe de l'aiguille 34 pénétrant dans la pièce perforable 80. Cette pièce perforable 80 peut être préperforée comme peut l'être le fond du capuchon 36. Pour guider l'aiguille lors de la mise en place de la seringue dans la pièce 14, il est prévu un tube de guidage 82 qui est disposé selon l'axe de la pièce 14 et qui est solidarisé avec celle-ci par l'intermédiaire d'ailettes rayonnantes telles que 84. Sur cette figure, on a également représenté un épaulement supplémentaire interne 85 qui permet de limiter l'enfoncement de la seringue dans la pièce 14 lorsqu'on procède à l'injection, dans le cas où le flacon est désolidarisé de la pièce de guidage 14, la seringue restant engagée dans la pièce de guidage.

En variante, il est possible de prévoir que la partie de la pièce de guidage 14 entourant la pièce perforable 80 présente un diamètre réduit par rapport à la partie courante de la pièce 14 de telle manière que la face latérale de la pièce perforable 80 soit pincée de façon étanche dans cette restriction assurant ainsi l'étanchéité à l'intérieur de la pièce de guidage 14 en complément du joint 48. En outre, on comprend que, la pièce perforable 80 étant plaquée avec pression contre la partie du bouchon 22 qui sera perforée par l'aiguille, cette partie du bouchon est protégée contre les risques de contamination. En outre, la pression ainsi appliquée sur le bouchon 22 améliore la qualité du perçage du bouchon.

On comprend que ces deuxième et troisième modes de réalisation peuvent comporter toutes les variantes décrites en liaison avec le premier mode de réalisation et notamment la préperforation du bouchon du flacon, la collerette de protection de la pièce de guidage, la fixation du bouchon sur le flacon à l'aide de la bride de clipsage et la présence d'un épaulement interne dans cette pièce de telle manière que, après enfoncement de l'aiguille, l'extrémité de celle-ci soit sensiblement dans le plan de la face interne du bouchon.

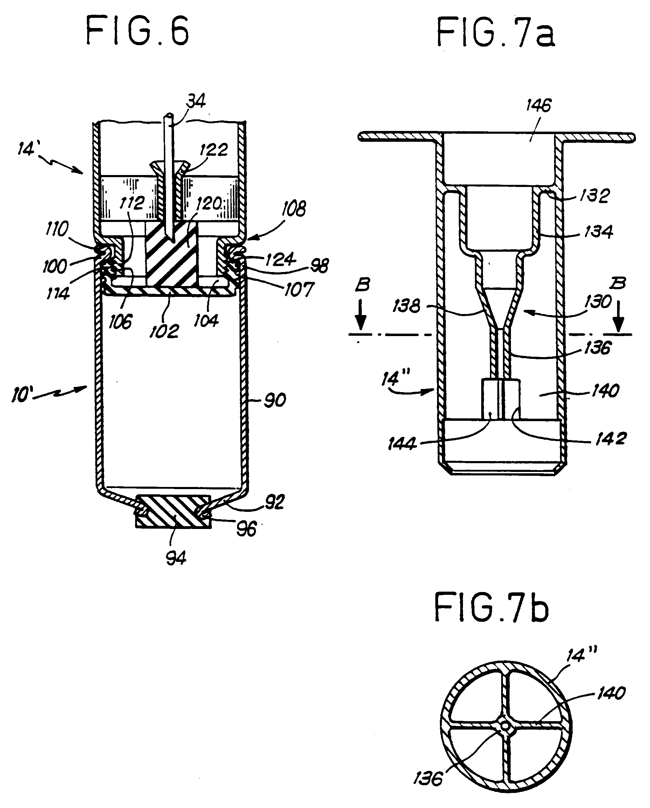

La figure 6 montre un quatrième mode de réalisation du dispositif de seringue qui se distingue essentiellement des précédents par la constitution du flacon 10 qui porte la référence 10' sur cette figure. Le flacon 10' comporte un corps 90, de préférence cylindrique, dont le fond 92 est obturé par une membrane perforable 94, dont la périphérie comporte une gorge qui coopère avec un bourrelet 96 du fond du flacon. La deuxième extrémité 98 du flacon est de préférence également munie d'un bourrelet 100. Cette extrémité 98 est obturée par un bouchon 102 réalisé en un matériau perforable, ou éventuellement préperforé. Le bouchon 102 est engagé en force dans le flacon au-delà du bourrelet 100. Le bouchon 102 comporte dans sa face supérieure un évidement 104, par exemple cylindrique. La paroi latérale de l'évidement 104 est muni d'un taraudage 106 dont la fonction sera explicitée ultérieurement. De préférence, la face latérale externe 107 du bouchon présente trois lèvres d'étanchéité avec la face interne du corps du flacon.

La pièce de guidage 14, qui porte sur cette figure la référence 14', est également modifiée par rapport aux pièces de guidage représentées sur les figures 1 à 5. L'extrémité 108 de raccordement de la pièce de guidage présente un épaulement externe 110 et elle se prolonge par une jupe 112 de diamètre réduit par rapport à celui de la partie courante de la pièce de guidage. Cette jupe 112 est munie sur sa face externe d'un filetage 114 qui peut coopérer avec le taraudage 106 du bouchon. Enfin, comme dans le cas de la figure 5, l'extrémité de l'aiguille est plantée dans une pièce perforable 120 dont l'extrémité supérieure est solidaire d'un tube de guidage 122 et dont l'extrémité inférieure est en appui sur le fond du bouchon 102.

Le mode d'utilisation du dispositif de seringue selon la figure 6 est le suivant :

Le flacon 10' est soumis aux opérations déjà décrites pour procéder à la lyophilisation du produit qu'il contient. Puis la pièce de guidage 14' avec sa seringue 12 remplie du liquide est fixée sur le flacon 10' par vissage de la jupe 112 de la pièce de guidage sur la face interne du bouchon 102 du flacon. L'épaulement 110 de la pièce de guidage vient en appui sur le bourrelet 100 du flacon tandis que le vissage tend à appliquer le bord libre supérieur 124 du bouchon sur la face interne de ce même bourrelet. La pièce de guidage est ainsi solidaire du flacon 10' et le bouchon 102 est également immobilisé en translation.

Puis on enfonce la seringue 12 dans la pièce de guidage 14'. La pointe de l'aiguille 34 perforé la pièce 120 et le bouchon 102 assurant ainsi la mise en communication de l'intérieur du flacon 10' avec l'intérieur de la seringue 12. En déplaçant le piston 52 de la seringue on fait passer le liquide qu'elle contenait à l'intérieur du flacon 10' et on procède au mélange du liquide et du produit lyophilisé.

On peut alors procéder au dévissage de la pièce de guidage 14' par rapport au bouchon 102 du flacon 10' qui devient autonome. Le mélange est ainsi contenu dans un flacon qui présente une extrémité munie d'une membrane perforable 94 et dont l'autre extrémité est obturée par un bouchon 102 qui peut servir de piston. Pour cela il suffit de visser une tige de commande sur le taraudage du bouchon 102.

Pour administrer à un patient le mélange contenu dans le flacon 10', il suffit de monter sur la membrane perforable 94 une aiguille à double pointe et d'enfoncer progressivement le bouchon 102 dans le flacon, le bouchon jouant le rôle de piston.

De même, le flacon 10' peut jouer le rôle de réservoir de liquide pour une pompe montée sur une tubulure raccordée au flacon via sa membrane perforable. Dans ce cas, le bouchon 102 va s'enfoncer dans le flacon sous l'effet de l'aspiration du liquide par la pompe.

Dans ce dernier mode de réalisation, on comprend que la fonction des pas de vis 106 et 114 est, d'une part, de solidariser temporairement le bouchon 102 sur le corps du flacon 10' à proximité de son ouverture 18 afin d'immobiliser le bouchon 102 et de permettre ainsi la perforation par la pointe de l'aiguille de la seringue et, d'autre part, de libérer en translation le bouchon 102, après enlèvement de la pièce de guidage 14' afin que ce dernier puisse jouer le rôle d'un piston. Il va de soi que l'on ne sortirait pas de la présente invention si le dispositif de seringue était muni d'autres moyens aptes à remplir cette double fonction. Par exemple le bouchon 102 pourrait être muni d'une bague externe prenant appui sur la périphérie de l'ouverture du flacon 10' empêchant ainsi l'enfoncement du bouchon dans le flacon. Après enlèvement de la pièce de guidage 14', cette bague serait brisée pour autoriser l'enfoncement du bouchon 102 qui pourrait alors jouer son rôle de piston.

On comprend également que le mode de réalisation du flacon décrit en liaison avec la figure 6 peut être combiné au mode de réalisation des figures 3 et 4, c'est-à-dire au cas où l'aiguille proprement dite est remplacée par la pièce tubulaire 60 présentant les pointes perforantes 62 et 64.

En se référant maintenant aux figures 7a et 7b, on va décrire une variante de réalisation de la pièce de guidage qui est alors référencéé 14''. A l'intérieur de la pièce 14'' est prévue une pièce de guidage 130, faisant partie intégrante de la pièce 14'' comprenant une collerette 132 de raccordement à la pièce 14'', une portion cylindrique 134 destinée à recevoir l'extrémité du corps de la seringue lorsque celle-ci est totalement enfoncée dans la pièce 14'' et une portion 136 de guidage de l'aiguille qui est raccordée à la portion cylindrique 134 par une portion sensiblement conique 138. Des ailettes radiales 140, mieux visibles sur la figure 7b, assurant le centrage de la partie de guidage 130. Les ailettes 140 présentent, à leurs extrémités inférieures des découpes 142 définissant un volume libre 144 destiné à recevoir la pièce perforable 80 dans laquelle la pointe de l'aiguille est enfoncée. La pièce perforable 80 est solidaire de la pièce de guidage 130. Enfin, au dessus de la collerette 138, la zone 146 de la pièce de guidage 14'' est destinée à recevoir le joint d'étanchéité 48.

Lorsque la pièce de guidage 14'' est montée sur le flacon la pièce perforable 80 est pincée entre le bouchon 22 du flacon et l'extrémité inférieure de la portion 136 de guidage. La pièce perforable obture donc de façon étanche l'extrémité inférieure de la partie de guidage 130 et elle protège de la contamination la partie centrale perforable du bouchon 22. De plus, lors de la mise en place de la seringue dans la pièce de guidage 14'', le joint 48 assure l'étanchéité à l'autre extrémité de la partie de guidage 130. Lors du stockage du dispositif de seringue, l'aiguille de la seringue est donc entièrement protégée à l'intérieur de la partie de guidage 130. Cette protection demeure pendant toutes les opérations de mélange des deux produits.

De préférence, la seringue est mise en place dans la pièce de guidage puis cet ensemble est fixé sur le flacon. Dans le cas du mode de réalisation des figures 7a et 7b, l'intérieur de la pièce 130 est obturée de façon étanche dès que le corps de la seringue coopère avec le joint 48. L'air contenu dans ce volume fermé peut contrarier l'enfoncement de la seringue jusqu'à ce qu'elle arrive dans sa position de stockage dans laquelle la pointe de l'aiguille pénètre dans la pièce 80. Pour permettre l'échappement de l'air durant cette phase, on peut prévoir la mise en place temporaire d'une deuxième aiguille qui traverse de part en part la pièce 80 et débouche à l'intérieur de la pièce 130. Cette deuxième aiguille peut ultérieurement servir à l'introduction dans la pièce 130 d'un agent gazeux de désinfection. Puis la deuxième aiguille est enlevée avant la fixation de la pièce 14'' sur le flacon. La pièce perforable étant un septum, l'orifice créé par l'introduction de l'aiguille se referme dès le retrait de celle-ci.

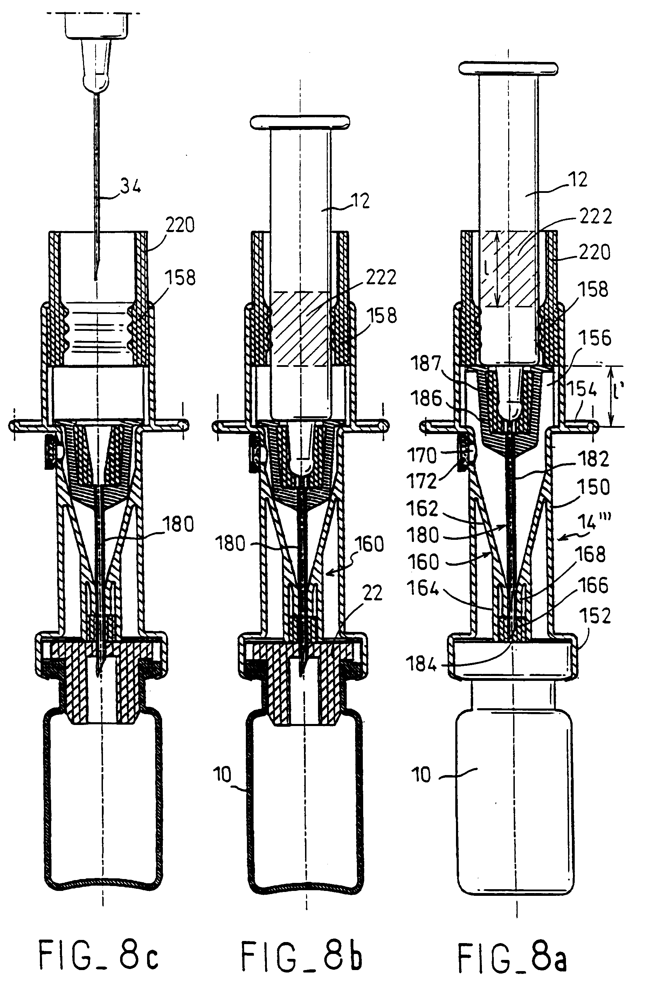

En se référant maintenant aux figures 8a à 8c, on va décrire une nouvelle variante de réalisation du dispositif de seringue. Selon ce mode de réalisation, le flacon 10 et la seringue 12 peuvent être identiques aux éléments correspondants représentés sur les figures 1 et 2 ou 5. On ne les redécrira donc pas.

La pièce de guidage 14''' comporte un corps cylindrique 150 dont une extrémité 152 forme un embout de fixation par clipsage sur le col 20 du flacon et la périphérie du bouchon 22. A sa deuxième extrémité le corps cylindrique est muni d'une collerette de protection 154 et il se prolonge par un logement 156 destiné à recevoir un joint d'étanchéité 158, de préférence à lèvre, qui peut être identique à celui de la figure 1. Comme on l'a déjà expliqué ce joint 158 assure l'étanchéité entre le corps de la seringue 12 et la pièce de guidage 14''' lors de tous les mouvements relatifs de la seringue par rapport à la pièce de guidage.

A l'intérieur du corps cylindrique 150 se trouve une structure de guidage 160 qui comprend une portion tronconique 162 solidaire du corps 150 prolongée par une portion terminale cylindrique 164. Dans la partie terminale 164 est montée une pièce perforable 166 réalisée en un matériau élastique. A l'intérieur de la portion cylindrique 164 est prévu un manchon 168 solidaire de la portion 166. L'extrémité libre du manchon appuie sur la face 166a de la pièce perforable 166. De plus, la deuxième face 166b de la pièce 166 fait légèrement saillie par rapport à l'extrémité libre de la portion cylindrique 164. Ainsi lorsque la pièce de guidage 14''' est fixée sur le flacon 10, la pièce perforable 166 est légèrement comprimée, ce qui assure un contact avec pression entre la face 166b de la pièce 166 et la partie centrale du bouchon 22 destinée à être perforée. Cette disposition peut également être prévue pour la pièce perforable 80 ou 120 des modes de réalisation des figures 5 à 7.

La figure 8a montre que le corps cylindrique de la pièce de guidage comporte un orifice 170 disposé au-dessus de la zone de raccordement de la structure de guidage 160 avec le corps cylindrique 150. L'orifice 170 est obturé par un filtre micro poreux 172, de type en soi connu. Le filtre 172 hydrophobe a des pores de dimensions très réduites capables de maintenir la stérilité du volume limité par la structure de guidage 160. Par exemple, les pores du filtre ont des dimensions de l'ordre de 0,22 microns.

Le filtre 172 a une double fonction. D'une part, il permet à l'air de s'échapper lors de l'enfoncement de la seringue 12 dans la pièce de guidage. Cette disposition est extrêmement souhaitable en raison de la présence du joint d'étanchéité 158. D'autre part, il permet, après la mise en place de la seringue dans la pièce de guidage 14''' et la fixation de celle-ci sur le flacon d'introduire dans la structure de guidage un gaz aseptisant. Il va de soi qu'un filtre micro poreux analogue au filtre 172 des figures 8 pourrait être monté sur les pièces de guidage 14 des figures 1 à 5, ou 14' et 14'' des figures 6 et 7.

La figure 8a montre également que, de préférence, l'aiguille 34 de la seringue est équipée d'un protège-aiguille 180. Le protège-aiguille 180 comprend un fourreau cylindrique 182 qui entoure l'aiguille 34. Sa première extrémité 184 est perforante et fait légèrement saillie au-delà de la pointe de l'aiguille. L'autre extrémité du fourreau 182 comprend une pièce de fixation amovible 186 sur l'extrémité d'injection du corps de la seringue 12. Lors de la mise en place de la seringue dans la pièce de guidage 14''', l'extrémité 184 du protège-aiguille 182 est guidée par le manchon 168 de la structure de guidage. Dans sa position finale de stockage, la pointe 184 du protège-aiguille perfore partiellement la pièce 166.

De préférence, la pièce de fixation 186 comporte une partie en matériau élastique 187 qui assure l'étanchéité entre l'extrémité de la seringue portant l'aiguille et le protège-aiguille 180.

Pour procéder au mélange des produits, comme on l'a déjà expliqué, on enfonce la seringue dans la pièce de guidage (fig. 8b). Durant ce déplacement, c'est la pointe 184 du protège-aiguille 182 qui perfore la pièce 166 et le bouchon 22 du flacon 10; protégeant ainsi la pointe de l'aiguille 34. Grâce au matériau dont il est constitué le protège-aiguille 182 reste coincé par frottement dans le bouchon 22 et la pièce 166 (fig. 8c) lorsqu'on enlève la seringue 12 de la pièce de guidage 14'''. Le maintien du protège-aiguille 182 peut également résulter du frottement entre la pièce de fixation 186 du protège-aiguille et la pièce de guidage 14''' ou encore d'une forme particulière donnée à cette dernière qui assure son clipsage sur la pièce de guidage.

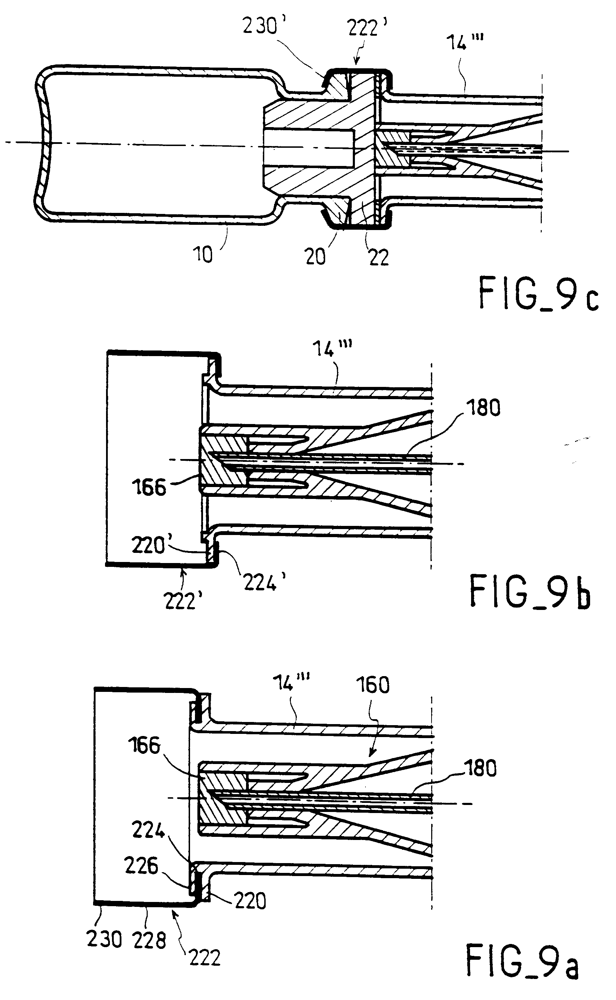

Dans les modes de réalisation décrits précédemment, la fixation de la pièce de guidage sur le flacon est obtenue grâce à une forme particulière de l'extrémité 28 de la pièce de guidage. Les figures 9a à 9c illustrent un autre mode de fixation.

Selon le mode de réalisation de la figure 9a, l'extrémité de la pièce de guidage 14''' est constituée par une collerette circulaire 220 destinée à venir en appui sur la périphérie du bouchon 22 du flacon. Une capsule de sertissage 222 en forme de bague comporte une première partie recourbée 224 qui est engagée dans une fente 226 de la collerette 220 et une deuxième partie cylindrique 228. Le bord libre 230 de la partie cylindrique 228 est destinée à être sertie sur la face inférieure du col 20 du flacon pour assurer la solidarisation de la pièce de guidage 14''' sur le flacon.

La figure 9b illustre une variante de montage de la capsule 222' de sertissage. Celle-ci comporte une portion repliée 224' qui repose sur la collerette 220' formant la première extrémité de la pièce de guidage 14'''. Le reste de la capsule de sertissage 222' est identique à celui de la capsule 222.

La figure 9c montre la fixation de la pièce de guidage 14''' de la figure 9b sur le flacon 10 muni de son bouchon 22. Le bouchon 22 peut déjà être fixé sur le col du flacon par une première capsule de fixation comme cela est montré sur la figure 1 ou bien ce bouchon peut être simplement enfoncé dans l'ouverture du flacon. Dans les deux cas, la pièce de guidage 14''', dans laquelle la seringue a déjà été mise en place, est appliquée contre le flacon de telle manière que la collerette 220' soit en appui contre la périphérie du bouchon 22. On procède alors au sertissage du bord libre 230' de la capsule de sertissage.

On comprend que la solution illustrée par les figures 9a à 9c permet de simplifier la solidarisation du flacon 10 et de la pièce et de la pièce de guidage 14''' puisque la capsule de sertissage 222 ou 222' déjà mise en place sur la pièce de guidage. Il va de soi que ce mode de solidarisation peut être appliqué au mode de réalisation décrit précédemment en modifiant la forme de la première extrémité de la pièce de guidage.

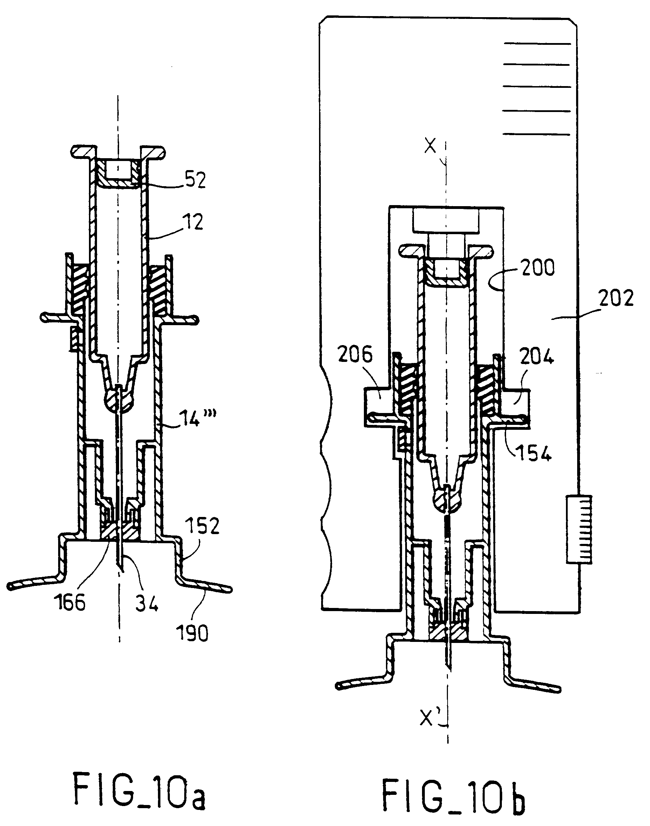

En se référant aux figures 10a à 10d, on va décrire l'utilisation d'un dispositif de seringue avec un auto-injecteur. Le dispositif de seringue est du type représenté sur les figures 8a à 8c à l'exception du fait qu'il ne comporte pas de protège-aiguille. En outre, de préférence, l'extrémité de clipsage 152 de la pièce de guidage 14''' se prolonge par une collerette 190 formant surface d'appui comme on l'expliquera ultérieurement.

Après avoir procédé au mélange des deux produits et à l'aspiration du mélange dans la seringue, comme on l'a expliqué précédemment, on désolidarise la pièce de guidage 14''' du flacon 10 et on enlève du piston 52 de la seringue 12 sa tige de commande. Le dispositif de seringue se trouve alors dans l'état représenté sur la figure 10a.

Dans l'étape suivante, représentée sur la figure 10b, on met en place le dispositif de seringue dans la cavité 200 d'un auto-injecteur 202. La seringue 12 est disposée selon l'axe XX' de la cavité de l'auto-injecteur et le dispositif de seringue est immobilisé en translation, par exemple, par la coopération de la collerette 154 avec des évidements 204 et 206.

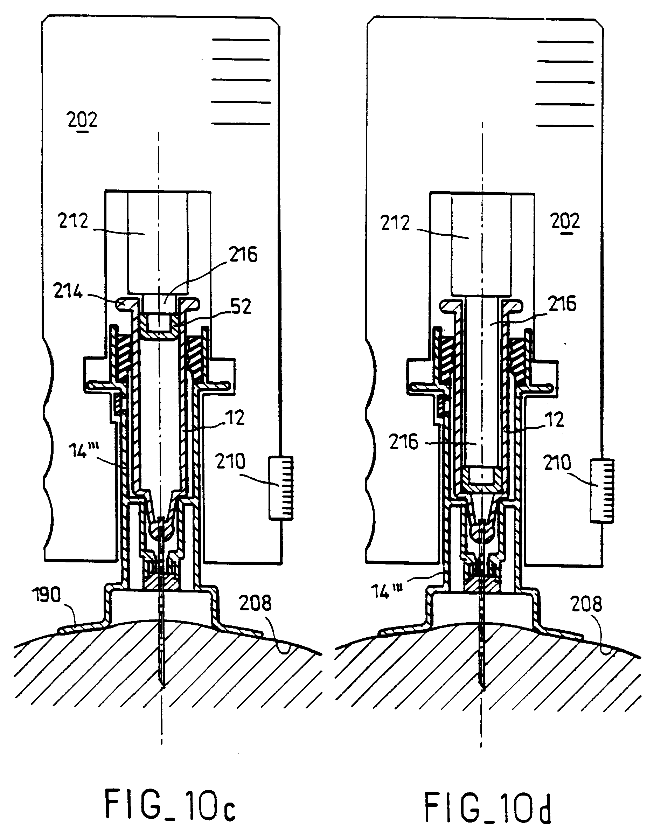

La figure 10c montre que l'ensemble ainsi préparé est mis en place sur le patient auquel une injection du mélange doit être faite. Plus précisément, la zone 190 est placée au contact de la partie 208 du corps du patient ou l'injection doit être pratiquée. En agissant sur le bouton de commande 210, on commande la sortie d'un premier poussoir 212 qui fait saillie dans la cavité 200 et qui prend appui sur l'extrémité ouverte 214 du corps de la seringue. Le déplacement du poussoir 212 provoque l'enfoncement de la seringue dans la pièce de guidage 14''' et donc l'enfoncement de l'aiguille 34 dans le corps du patient d'une longueur prédéterminée. Durant cette opération, le piston 52 de la seringue n'est pas déplacé par rapport au corps de la seringue.

Dans l'ultime phase opératoire représentée sur la figure 10d, on provoque l'actionnement d'un deuxième poussoir 216 coaxial au poussoir 212, le poussoir 212 restant immobile. le déplacement du poussoir 216 entraîne l'enfoncement du piston 52 de la seringue à une vitesse contrôlée jusqu'à ce que la totalité du mélange contenu dans le corps de la seringue ait été injectée au patient. De préférence, l'actionnement du bouton de commande 210 provoque la commande successivement du poussoir 212 et du poussoir 216.

On comprend que, lorsque l'opérateur manipule le dispositif de seringue en vue d'enfoncer la seringue dans le tube de guidage pour provoquer la perforation du bouchon du flacon, la face externe du corps de seringue qui est extérieure au joint d'étanchéité du tube de guidage risque d'être contaminée, en particulier par les mains de l'opérateur. Or une partie de cette surface, qui est hachurée sur la figure 8a, va pénétrer dans la chambre étanche et stérile définie par le tube de guidage et le joint d'étanchéité. Cela risque d'entraîner la contamination de cette chambre et des parties du dispositif qu'elle contient.

Les figures 8a à 8c illustrent un premier mode de réalisation de ces moyens de protection. Ils sont constitués par une jupe cylindrique 220 qui s'étend au-delà de l'extrémité externe du joint d'étanchéité 158. Le diamètre interne de cette jupe est supérieur au diamètre externe du corps de la seringue. La longueur axiale l de la jupe est au moins égale à la course l' de la seringue dans le tube du guidage 14''' entre sa position de stockage (fig. 8a) et sa position de perforation (fig. 8b). On comprend qu'ainsi la zone hachurée 222 de la surface externe de la seringue, destinée à pénétrer dans le tube, ne peut être touchée par l'opérateur, ce qui évite les risques de contamination par contact de cette zone.

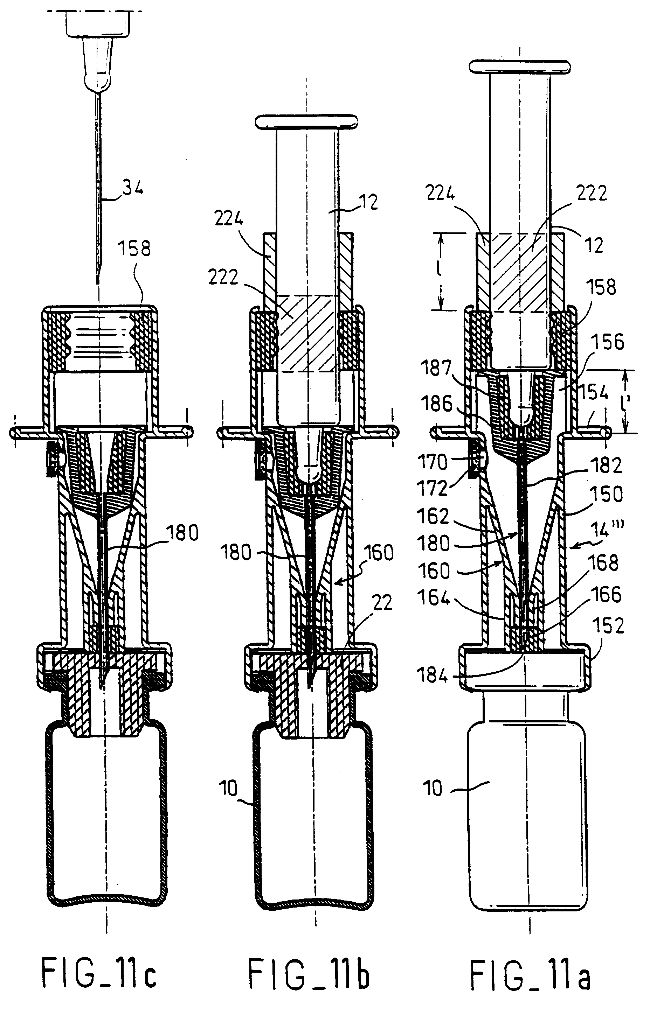

Les figures 11a à 11c illustrent un deuxième mode de réalisation des moyens annulaires de protection. Ces moyens sont constitués par un manchon élastique 224 qui est monté sur la portion du corps de seringue adjacente à l'extrémité du joint 158 lorsque la seringue est montée dans le tube de guidage. La zone hachurée est donc protégée contre tout risque de contamination par contact ou par l'air ambiant. Le manchon 224 a une longueur l qui est au moins égale à la course l' de la course du corps de seringue dans le tube de guidage. Lors de l'enfoncement de la seringue dans le tube, le joint d'étanchéité constitue une butée pour le manchon de protection 22', ce dernier glissant par rapport au corps de seringue.

Il va de soi que les moyens annulaires de protection qui viennent d'être décrits en liaison avec les figures 8 et 11 pourraient être utilisés avec les modes de réalisation décrits en référence aux figures 1 à 7 et 9.

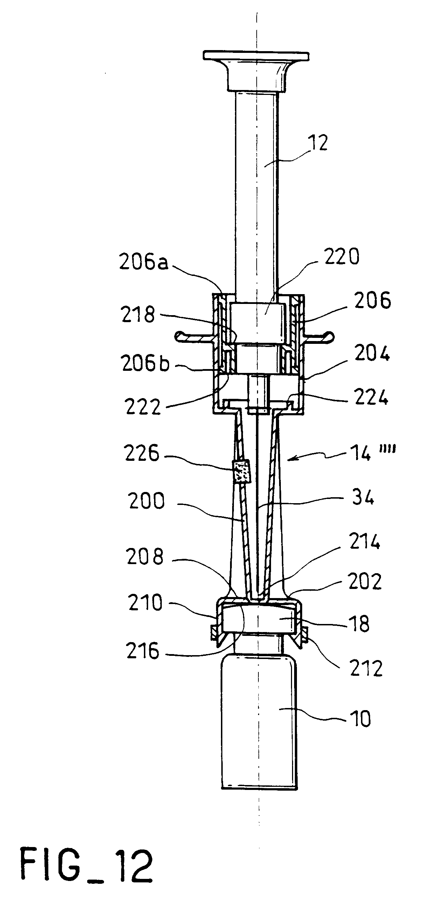

La figure 12 illustre un autre mode de réalisation du dispositif de seringue qui permet de protéger l'ensemble contre des risques de contamination liés à la saisie du corps de seringue par l'utilisateur. La pièce de guidage 14''' comporte une partie courante 200 de forme tronconique, une extrémité 202 de fixation sur le flacon 10 et une extrémité de liaison 204 pour recevoir un joint d'étanchéité 206 et le corps de seringue 12. L'extrémité de fixation comprend une collerette 208 et des organes 210 de clipsage sur l'ouverture du flacon. Les pattes de clipsage peuvent être complétées par une bague de sertissage 212. Une fois mise en place, la bague 212 peut pénétrer dans une gorge ménagée dans les pattes de clipsage assurant ainsi le verrouillage de la pièce de guidage sur le flacon. La collerette 208 laisse libre une zone centrale 214 pour le passage de l'aiguille 34. De préférence, le passage 34 est entouré par une nervure circulaire 216 qui fait saillie en-dessous de la collerette 208. Cette nervure coopère avec le bouchon du flacon 10 pour assurer une étanchéité entre les deux pièces.

L'extrémité supérieure 204 de la pièce de guidage présente une forme cylindrique élargie dans laquelle est monté le joint annulaire 206. Ce joint peut être en caoutchouc (Butyle) ou en matériau plastique. Le joint 206 présente un épaulement 218 qui coopère de façon étanche avec une portion élargie 220 du corps de seringue. Le joint 206 présente sur sa face externe deux bourrelets d'étanchéité 206a et 206b qui assurent l'étanchéité dynamique tout en permettant le glissement du joint sous l'effet d'une poussée. Sur sa tranche inférieure, le joint comporte de préférence un évidement annulaire 222 qui coopère avec une nervure de clipsage 224 lorsque la seringue est totalement enfoncée dans la pièce de guidage.

On comprend que l'intérieur de la pièce de guidage contenant l'aiguille 34 est étanche, notamment vis-à-vis des germes de contamination du fait du joint 206 et de la nervure d'étanchéité 216. De plus, la zone centrale du bouchon qui sera perforée par l'aiguille est également protégée vis-à-vis des risques de contamination.

Le mode d'utilisation du dispositif de seringue de la figure 12 est le suivant : en position de stockage, représentée sur la figure 12, le joint 206 est en position non enfoncée dans la pièce de guidage. Lorsqu'on veut procéder au mélange du produit contenu dans le flacon 10 avec le liquide contenu dans la seringue, on enfonce la seringue dans la pièce de guidage 14''', ce qui provoque l'enfoncement simultané du joint 206 grâce à la coopération de l'épaulement 218 et de la partie 220 du corps de seringue. A la fin de l'enfoncement de la seringue, les éléments de clipsage 222 et 224 coopèrent et assurent la solidarisation du joint sur la pièce de guidage. Après avoir rempli la seringue avec le mélange, il suffit d'extraire celle-ci du joint qui reste solidaire de la pièce de guidage.

Il est important d'observer que, grâce au déplacement du joint 206 provoqué par l'enfoncement de la seringue, la partie de la seringue extérieure à la pièce de guidage en position de stockage, qui est susceptible d'être contaminée par l'utilisateur, reste externe à la zone étanche interne à la pièce de guidage limitée par la position du joint 206. Il n'y a donc, dans la phase d'enfoncement, aucun risque de contamination de l'aiguille.

Il peut être intéressant d'équiper la partie courante de la pièce de guidage d'un orifice obstrué par un filtre bactériologique, l'ensemble étant figuré par la référence 226. Ce filtre autorise la sortie d'air lors de l'enfoncement de la seringue dans la pièce de guidage, tout en maintenant l'asepsie de l'espace contenant l'aiguille 34.

Cependant, comme le joint 206 est coulissant et est maintenu en position enfoncée après l'extraction de la seringue, la surpression lors de la réintroduction de la seringue est très réduite. Le filtre est donc moins indispensable dans cette fonction.

Plusieurs autres variantes de réalisation peuvent être prévues dans le cadre de l'invention. En particulier, il est possible de prévoir que, lors du retrait de la seringue par rapport à la pièce de guidage, l'aiguille se désolidarise de l'extrémité de la seringue en restant plantée, par exemple, dans la pièce perforable ou dans le capuchon.

Selon une autre variante de réalisation, on peut modifier la forme du joint d'étanchéité entre la pièce de guidage et le corps de seringue pour faciliter la séparation de la seringue par rapport à la pièce de guidage. Pour cela on ménage un filetage dans la face externe du joint, ce filetage coopérant avec un taraudage ménagé dans la face interne de la pièce de guidage. La face interne du joint reste munie de ses lèvres d'étanchéité. Pour séparer la seringue de la pièce de guidage il suffit de faire tourner le corps de la seringue ce qui entraîne le dévissage du joint. Bien entendu on pourrait utiliser d'autres moyens de solidarisation amovible du joint sur l'extrémité de la pièce de guidage.

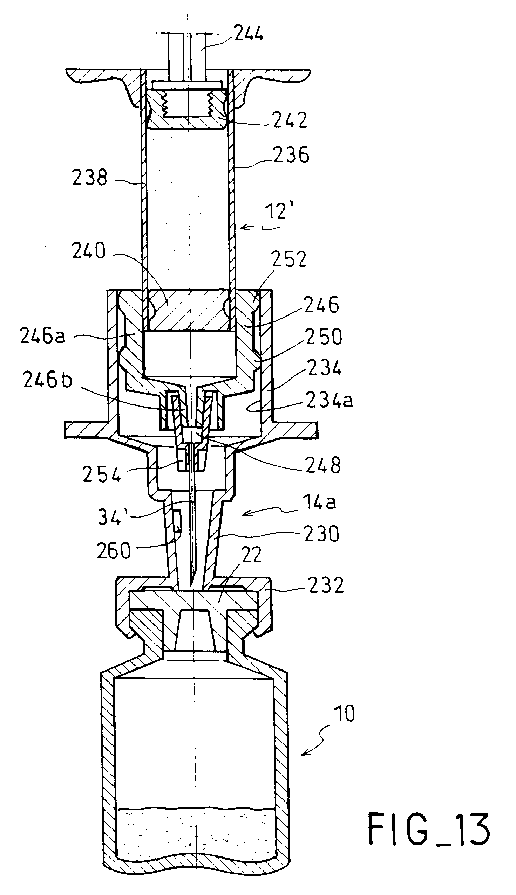

La figure 13 illustre un autre mode de réalisation du dispositif de seringue montrant en particulier une autre façon de réaliser l'étanchéité entre le corps de seringue et la pièce de guidage.

Le dispositif de seringue comporte un flacon 10 destiné à recevoir un produit, notamment le produit lyophilisé, qui est fermé par un bouchon perforable 22. Sur le flacon est fixée une pièce de guidage portant la référence 14a. Cette pièce de guidage comporte une portion tronconique 230 prolongée par une collerette 232 de fixation sur le bouchon et sur le col du flacon 10. A l'autre extrémité de la portion tronconique 230 est prévue une portion cylindrique 234 servant effectivement au guidage de la seringue qui porte ici la référence 12'. Dans ce mode de réalisation particulier, la seringue 12' consiste en une carpule 236 constituée de façon classique par un corps en verre cylindrique 238 fermé à sa première extrémité par un bouchon 240 et à sa deuxième extrémité par un autre bouchon formant piston 242. Le piston 242 peut recevoir l'extrémité de la tige de commande de la seringue 244. A la première extrémité de la carpule 238 est fixé un embout cylindrique 246 qui est solidaire de l'extrémité de la carpule. L'embout 246 comporte une partie cylindrique 246a et une extrémité tronconique 246b dans laquelle est vissée l'extrémité de fixation 248 d'une aiguille 34'. Comme le montre la figure 13, la partie cylindrique 246a de l'embout 246 est munie sur sa face externe de deux nervures annulaires 250 et 252 formant lèvres d'étanchéité, ces nervures faisant saillie hors de la paroi externe de l'embout et coopérant avec la face interne 234a de la partie cylindrique de la pièce de guidage. On comprend que les deux nervures ou lèvres 250 et 252 forment un joint d'étanchéité dynamique entre l'embout 246 et donc la seringue 12' et la partie 234 de la pièce de guidage 14a. Cette étanchéité est maintenue durant la phase d'enfoncement de la seringue 12' pour perforer le bouchon 22 du flacon 10. On comprend que les nervures 250 et 252 constituent ainsi dans ce mode de réalisation les premiers moyens d'étanchéité entre le corps de seringue 12' et la pièce de guidage 14a.

De préférence, la tête 248 de l'aiguille 34' comporte des nervures rayonnantes telles que 254. A l'intérieur de la partie tronconique 230 de la pièce de guidage est prévue une portion 260 formant saillie à l'intérieur de la pièce de guidage. Cette portion 260 se retrouve au niveau des nervures 254 de l'aiguille 34' lorsque celle-ci a été enfoncée pour provoquer la perforation du bouchon 22. On comprend que, pour désolidariser l'aiguille 34' de l'embout 246 en toute sécurité, il suffit à l'utilisateur, lorsque la seringue 12' est en position enfoncée, de provoquer la rotation de celle-ci. L'élément en saillie 260 étant interposé entre les nervures 254 de la tête de l'aiguille, le mouvement de rotation provoque le dévissage automatique de l'aiguille sans que l'utilisateur ait à toucher celle-ci.

On comprend également qu'on ne sortirait pas de l'invention si le corps de seringue 12' était constitué par une seringue de type classique réalisée en un matériau plastique légèrement déformable. Dans ce cas, les nervures annulaires d'étanchéité 250 et 252 seraient directement réalisées sur la face externe du corps de seringue. De même, dans ce cas, les moyens automatiques de dévissage de l'aiguille pourraient être prévus.

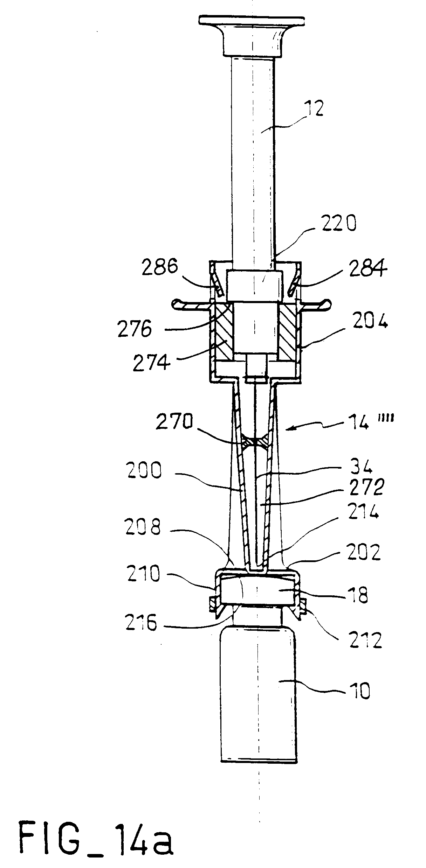

La figure 14a illustre une variante de réalisation de la seringue de la figure 12. Dans ce mode de réalisation, la pièce de guidage 200 est munie d'une membrane élastique étanche perforable 270 qui définit avec le bouchon perforable du flacon 10 un volume étanche 272. En position de stockage, l'aiguille 34 traverse de façon étanche la membrane 270. La membrane 270 sert à confiner dans le volume 272 la fraction de liquide résiduel ou de gaz qui pourrait sortir du flacon 10 lorsqu'on retire la pointe de l'aiguille 34 hors du flacon du fait de la déformation temporaire et locale du bouchon perforable dans la zone de perforation et de la surpression existant éventuellement dans le flacon après le mélange. Grâce à son élasticité, la membrane tend à se refermer dès que l'aiguille ne la traverse plus. Cette disposition de chambre de confinement de gaz peut être prévue dans tous les modes de réalisation du dispositif de seringue.

Sur la figure 14a, on a également représenté un nouveau mode de réalisation du joint d'étanchéité entre la partie supérieure 204 de la pièce de guidage et le corps de seringue. Le joint annulaire 274 comporte une face interne serrée sur la seringue 12 et coopère avec un épaulement 276 de la seringue de telle manière que l'enfoncement du corps de seringue provoque également l'enfoncement du joint. La partie supérieure 206 de la pièce de guidage comporte deux petites languettes élastiques 284 et 286 escamotables qui font saillie à l'intérieur de la pièce de guidage. En position de stockage, représentée sur la figure 14a, les languettes 284 et 286 font saillie entre la lèvre supérieure 282 et la lèvre intermédiaire 280. Cela empêche donc une sortie accidentelle du joint 274 hors de la pièce de guidage ou un enfoncement accidentel de ce joint.

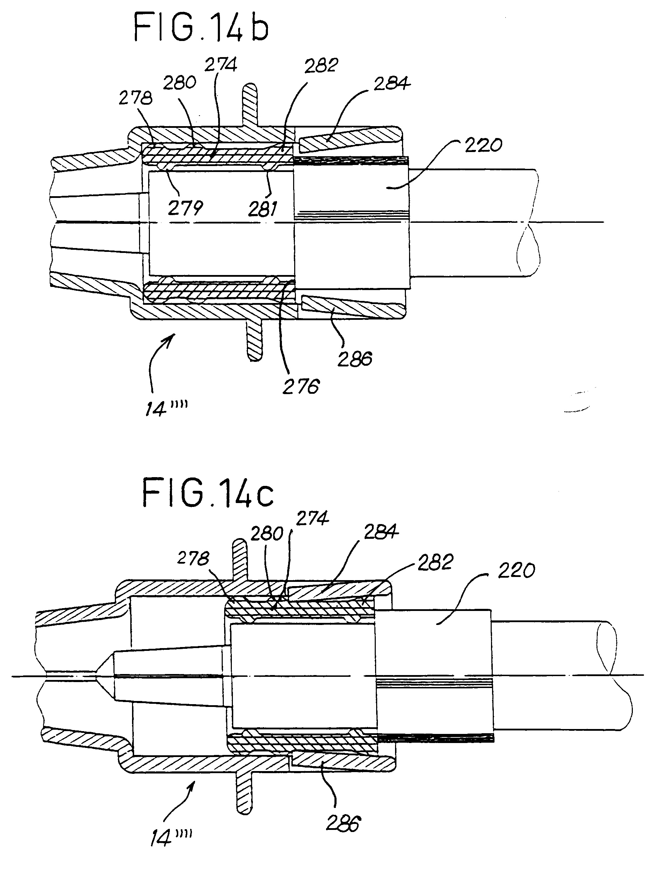

Les figures 14b et 14c montrent plus en détails le joint 274 et son mode d'action. Le joint présente de préférence trois lèvres externes d'étanchéité 278, 280 et 282 en contact avec la face interne de la pièce de guidage et deux lèvres internes 279 et 281 en contact avec le corps de seringue. En position de stockage (figure 14a) le joint est en position haute et les languettes sont engagées entre la lèvre supérieure 282 et la lèvre médiane 280. Les languettes 284 et 286 jouent le rôle de butées anti-retour autorisant seulement l'enfoncement du joint 274 et en empêchant sa sortie.

Dans cette position, l'étanchéité est assurée par la lèvre médiane 280 et par la lèvre inférieure 278. En outre, la coopération des languettes 284, 286 avec la lèvre intermédiaire 280 interdit une sortie accidentelle du joint 274 hors de la pièce de guidage.

Lors de l'enfoncement de la seringue pour perforer le bouchon du flacon, le joint 274 est entraîné par le corps de seringue et la lèvre supérieure 282 provoque une déformation temporaire des languettes de telle manière que la lèvre passe "en-dessous" des languettes. Les languettes, par leur fonction anti-retour, empêchent le déplacement du joint lors de l'extraction de la seringue hors de la pièce de guidage. Le joint est maintenu en position enfoncée.

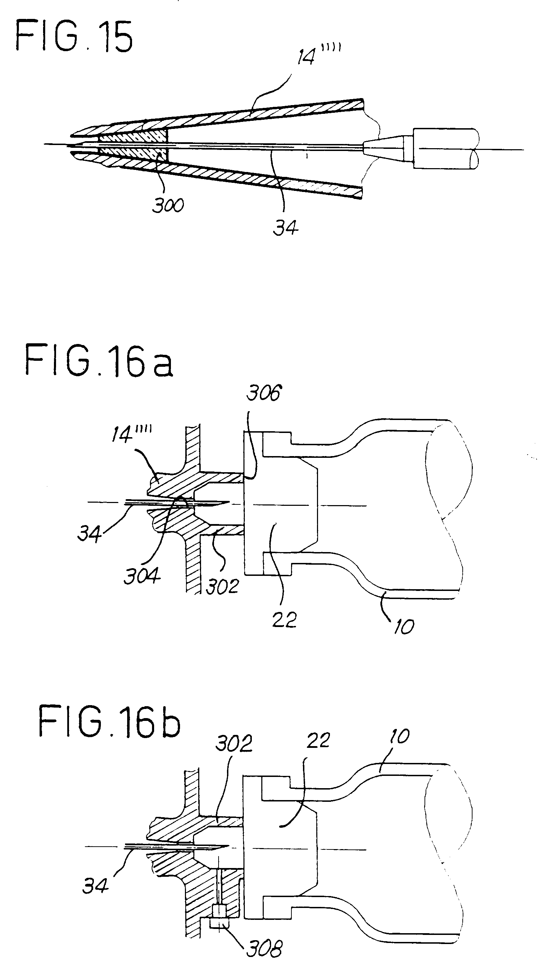

La figure 15 illustre un autre mode de réalisation du confinement des gaz nocifs susceptibles de sortir du flacon. Il consiste à disposer à l'extrémité de la partie tronconique de la pièce de guidage 14'''' une pièce 300 en matériau poreux tel qu'une mousse poreuse. Lors de l'extraction de l'aiguille hors du flacon, les gaz sortant éventuellement de ce dernier sont absorbés, piégés ou bloqués par la pièce 300. Bien entendu, la pièce 300 est traversée par l'aiguille 34.

Les figures 16a et 16b montrent deux autres modes de réalisation de la chambre de confinement des gaz. L'extrémité inférieure de la partie conique de la pièce de guidage est prolongée par un logement 302 raccordé à la pièce de guidage par un passage 304 dont le diamètre est sensiblement égal à celui de l'aiguille 34. La paroi du logement 302 se termine par un bord libre 306 qui est appliqué de façon étanche sur le bouchon 22 du flacon. On réalise ainsi une chambre sensiblement étanche dans laquelle les gaz sortant éventuellement du flacon sont confinés.

Dans le cas où le mélange à injecter est susceptible de produire une quantité importante de gaz, il est intéressant de munir la paroi du logement 302 d'un filtre microporeux 308 permettant de compenser la surpression créée grâce à la sortie des gaz filtrés hors du logement 302 tout en filtrant la partie toxique des gaz.

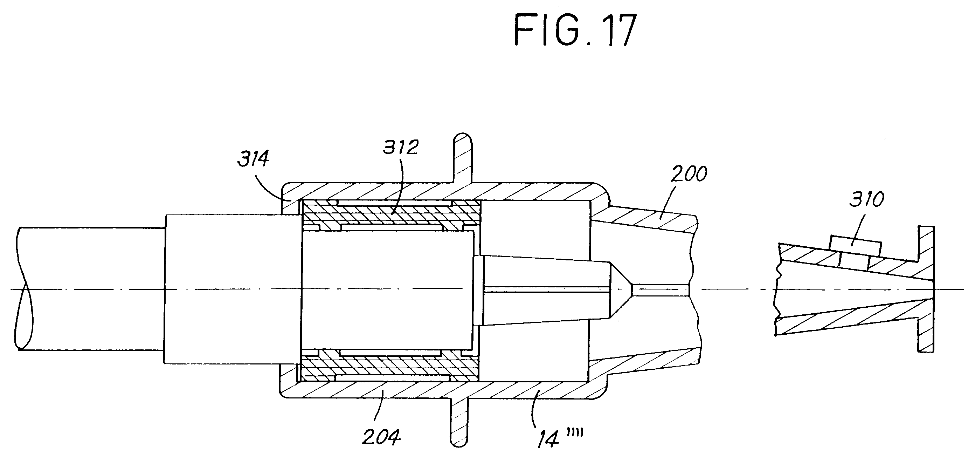

La figure 17 illustre un autre mode de récupération des gaz susceptibles de sortir du flacon lors de l'extraction de l'aiguille. La partie conique 200 de la pièce de guidage 14'''' est munie d'un filtre microporeux 310 permettant l'évacuation contrôlée du gaz. Pour permettre l'évacuation du gaz tout en maintenant l'étanchéité de la pièce de guidage 14'''', le joint d'étanchéité 312 qui comporte des lèvres d'étanchéité internes et externes entre le corps de seringue et la pièce de guidage n'est retenu dans la pièce de guidage que par un épaulement supérieur 314 empêchant la sortie du joint, la paroi latérale de la pièce de guidage étant elle-même étanche. Ainsi durant toute la phase d'extraction de la seringue, l'intérieur de la pièce de guidage reste étanche par la coopération du joint 312 et de la seringue. En conséquence, le gaz a le temps de s'échapper par le filtre 310 pour compenser la surpression de gaz dans la pièce de guidage tout en filtrant les gaz toxiques à l'aide du filtre 310.

Après le mélange des produits, l'opérateur doit procéder à l'extraction de la seringue hors de la pièce de guidage. Compte tenu de la nature des matériaux constituant la pièce de guidage, le joint d'étanchéité et la seringue, des forces de frottement importantes peuvent intervenir. Il en résulte que l'opérateur doit exercer une certaine force sur la seringue par rapport à la pièce de guidage pour libérer celle-ci. Durant cette opération, on ne peut donc exclure que, par un mouvement intempestif de l'opérateur, l'aiguille ne pique la main de l'opérateur tenant la pièce de guidage. Pour éviter ce risque, selon un mode perfectioné de réalisation du dispositif de seringue, l'extraction de la seringue se fait en deux temps. Dans un premier temps, la seringue est partiellement extraite de la pièce de guidage, l'essentiel des forces résistantes dues notamment aux frottements étant vaincues. Dans un deuxième temps, après une manipulation particulière, la seringue est totalement libérée de la pièce de guidage sans qu'il y ait de forces de traction importantes à exercer par l'utilisateur. Pour obtenir ce résultat, il est prévu une butée mécanique qui limite l'extraction de la seringue lors de la première phase d'extraction, l'extraction finale ne pouvant être obtenue qu'après rotation de la seringue par rapport à la pièce de guidage.

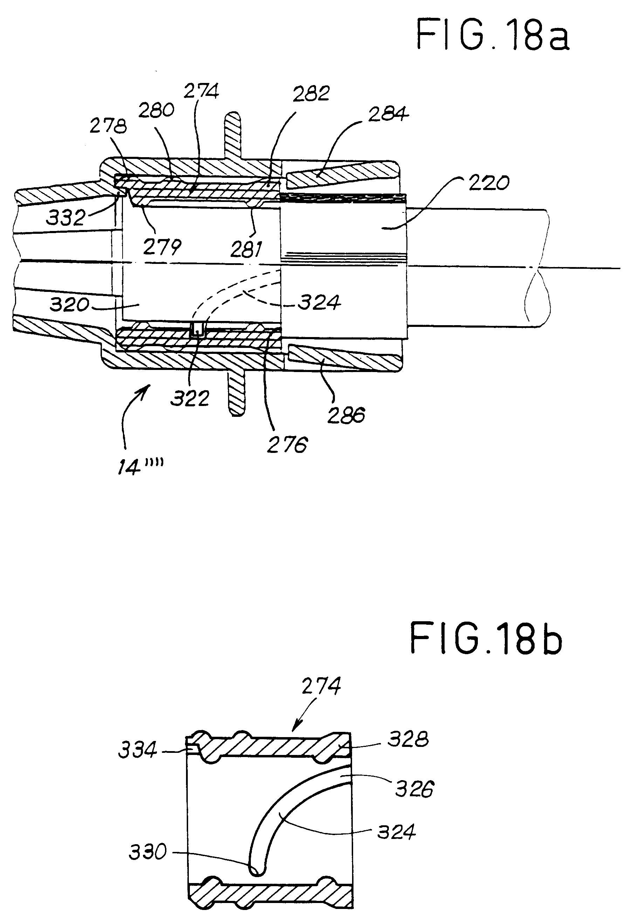

Les figures 18a et 18b illustrent cette disposition dans le cas du mode de réalisations des figures 14. Il va cependant de soi que cette disposition pourrait être aisément transposée aux autres modes de réalisation du dispositif de seringue.

Comme le montre la figure 18a, l'extrémité 320 de la seringue est munie d'un ergot 322. L'ergot 322 est engagé dans une rainure hélicoïdale 324 ménagée dans la face interne du joint 274. L'extrémité 326 de la rainure 324 débouche dans la face supérieure 328 du joint ; en revanche son autre extrémité est borgne. La rainure 324 correspond, par exemple, à une rotation de 90 degrés. On comprend que cette rainure interne ne rompt donc pas l'étanchéité procurée par le joint 274. En outre, un ergot 332 faisant saillie hors de la pièce de guidage coopère avec un évidement 334 du joint immobilisant celui-ci en rotation. En position de stockage et lors de l'opération de mélange des produits, l'ergot 322 est en butée sur l'extrémité borgne 330 de la rainure. Lorsque l'utilisateur exerce une force de traction sur la seringue, les forces de frottement sont libérées et la seringue reste solidaire du joint 274 et donc de la pièce de guidage. Ce n'est qu'après avoir provoqué une rotation de la seringue de 90 degrés par rapport à la pièce de guidage que l'ergot 322 se trouve en regard de l'extrémité ouverte 326 de la rainure et que la seringue peut être totalement extraite. Cette extraction est opérée sans avoir à exercer d'effort significatif et le mouvement de l'opérateur peut donc être parfaitement contrôlé.

Bien entendu d'autres solutions pourraient être envisagées pour n'autoriser la sortie de la seringue qu'en deux temps séparés par une opération de rotation de la seringue.

Comme cela a déjà été mentionné, de préférence le dispositif de seringue comprend la seringue avec sa pièce de guidage et le flacon avec son bouchon perforable. Cependant on ne sortirait pas de l'invention si l'on fournissait séparément la seringue avec sa pièce de guidage et le flacon, les deux composants étant solidarisés ultérieurement pour reconstituer un ensemble complet. Dans ce cas, il va de soi que, durant la période de stockage, la première extrémité de la pièce de guidage doit être obturée de façon étanche pour garantir l'aseptie de l'aiguille. Une autre solution consisterait à stériliser la pièce de guidage et l'extrémité de la seringue avec son aiguille immédiatement après son montage sur le flacon.

L'homme de l'art comprend également que les premiers moyens d'étanchéité entre la seringue et la pièce de guidage pourraient également consister en un joint monté entre ces deux éléments et consistant en un soufflet d'étanchéité monté d'une part sur la seringue et d'autre part sur la pièce de guidage. Les déformations du soufflet absorbent alors les déplacements relatifs de la seringue et de la pièce de guidage durant les opérations de mélange.

## Revendications

1. Dispositif de seringue permettant le mélange de deux composés dont au moins le premier est liquide, comprenant :
une seringue (12) présentant un corps cylindrique muni d'une extrémité d'injection (32), la seringue contenant ledit premier composé, comprenant en outre :
des moyens (14, 14', 14'', 14''', 14'''') de guidage en translation de l'extrémité d'injection de ladite seringue entre une première position en retrait dite de stockage et une deuxième position enfoncée dite active, lesdits moyens de guidage comportant une face interne, une première extrémité destinée à déboucher en regard de la zone perforable d'un bouchon apte à obturer de façon étanche l'ouverture d'un flacon destiné à contenir un desdits composés et une deuxième extrémité (46, 204) pour recevoir l'extrémité d'injection de ladite seringue, lesdits moyens de guidage étant étanches entre leur première et deuxième extrémité, ladite deuxième extrémité comportant des moyens d'étanchéité et des moyens pour assurer une solidarisation suffisante entre ladite seringue et lesdits moyens de guidage tout en autorisant ladite translation; et
des moyens de mise en communication (34, 60) disposés, selon l'axe du corps de ladite seringue pour permettre la perforation de ladite zone perforable dudit bouchon et la mise en communication du volume interne de ladite seringue avec l'intérieur dudit flacon lorsque l'extrémité d'injection de ladite seringue est amenée dans sa deuxième position,
ledit dispositif se caractérisant en ce que lesdits moyens d'étanchéité incluent un joint (48, 158, 206, 246, 274) monté à l'intérieur desdits moyens de guidage, ledit joint étant apte à assurer une étanchéité entre le corps de la seringue et la face interne des moyens de guidage en position de stockage et lors des déplacements relatifs des deux pièces.

2. Dispositif de seringue selon la revendication 1, caractérisé en ce qu'il comprend ledit falcon muni du bouchon et en ce qu'il comprend en outre des moyens de solidarisation (28, 108, 222, 222') de ladite première extrémité des moyens de guidage sur l'ouverture (18) dudit flacon (10, 10').

3. Dispositif de seringue selon la revendication 2, caractérisé en ce que ledit flacon (10) est muni d'un fond.

4. Dispositif de seringue selon la revendication 3, caractérisé en ce que les moyens de solidarisation (28) de la pièce de guidage (14) sur ledit flacon comprennent en outre des moyens de solidarisation étanche de la périphérie dudit bouchon (22) sur ledit flacon (10).

5. Dispositif de seringue selon l'une quelconque des revendications 1 à 4, caractérisé en ce que lesdits moyens de solidarisation (28, 222, 222') peuvent être rompus pour permettre la désolidarisation du flacon (10) par rapport à la pièce de guidage (14, 14', 14'', 14''').

6. Dispositif de seringue selon l'une quelconque des revendications 3 à 5, caractérisé en ce que lesdits moyens de solidarisation comprennent une capsule de sertissage (222, 222') montée sur ladite première extrémité (220, 220') desdits moyens de guidage (14''') et apte à être sertie sur une collerette (20) de l'ouverture dudit flacon (10) lors de la fixation desdits moyens de guidage sur ledit flacon.

7. Dispositif de seringue selon l'une quelconque des revendications 3 à 5, caractérisé en ce que lesdits moyens de fixation comprennent des pattes de clipsage coopérant avec la périphérie de l'ouverture du flacon.

8. Dispositif de seringue selon la revendication 7, caractérisé en ce que lesdits moyens de clipsage comprennent en outre une bague de sertissage coopérant avec les pattes de clipsage.

9. Dispositif de seringue selon l'une quelconque des revendications 1 à 8, caractérisé en ce que les moyens de mise en communication comprennent une aiguille (34) montée à l'extrémité d'injection de la seringue et dont la pointe est apte à perforer la zone centrale du bouchon (22) lorsque l'extrémité de la seringue est amenée dans sa deuxième position.

10. Dispositif de seringue selon l'une quelconque des revendications 1 à 9, caractérisé en ce que les moyens de mise en communication comprennent une pièce tubulaire (60) dont une première extrémité (64) est apte à obturer la zone centrale du bouchon (22) et dont l'autre extrémité (62) est apte à percer une cloison perforable (70) ménagée à l'extrémité d'injection (32) de la seringue (12) lorsque celle-ci est amenée dans sa deuxième position.

11. Dispositif de seringue selon la revendication 10, caractérisé en ce que ladite pièce tubulaire est constituée par deux aiguilles entre lesquelles est disposé un filtre.

12. Dispositif de seringue selon la revendication 9, caractérisé en ce qu'il comprend en outre un capuchon (36) entourant ladite aiguille (34) et dont une extrémité est perçable par la pointe de ladite aiguille lorsque l'extrémité de ladite seringue est amenée dans sa deuxième position.

13. Dispositif de seringue selon la revendication 12, caractérisé en ce que ledit capuchon (36) est réalisé en un matériau élastique déformable et en ce qu'il s'étend sur toute la longueur de l'aiguille lorsque l'extrémité d'injection (32) de la seringue est dans sa première position.

14. Dispositif de seringue selon l'une quelconque des revendications 12 et 13, caractérisé en ce que ledit capuchon (36) comprend des moyens d'immobilisation (42) en translation à l'intérieur desdits moyens de guidage (14).

15. Dispositif de seringue selon la revendication 9, caractérisé en ce qu'il comprend en outre une pièce perforable (80, 120) solidaire des moyens de guidage (14) et dans laquelle l'extrémité de l'aiguille (34) est enfoncée.

16. Dispositif de seringue selon la revendication 15, caractérisé en ce que ladite pièce perforable (80, 120) est préperforée.

17. Dispositif de seringue selon l'une quelconque des revendications 15 et 16, caractérisé en ce que ladite pièce perforable est réalisée en un matériau élastique et en ce que la face de ladite pièce destinée à être appliquée contre le bouchon dudit flacon est disposée par rapport auxdits moyens de guidage de telle manière que, lorsque ladite pièce de guidage est fixée sur ledit flacon, ladite pièce perforable est comprimée.

18. Dispositif de seringue selon l'une quelconque des revendications 9, 12 à 15, caractérisé en ce qu'il comprend en outre un tube axial de guidage (82) solidaire desdits moyens de guidage (14) et entourant l'aiguille lorsque la seringue est mise en place dans les moyens de guidage.

19. Dispositif de seringue selon l'une quelconque des revendications 1 à 18, caractérisé en ce que lesdits moyens de guidage (14) présentent un premier épaulement interne (49) pour limiter l'enfoncement du corps de la seringue dans lesdits moyens de guidage, de telle manière que l'extrémité de l'aiguille ne perfore par le bouchon (22) en situation de stockage.

20. Dispositif de seringue selon l'une quelconque des revendications 1 à 19, caractérisé en ce que lesdits moyens de guidage comprennent des moyens (50) pour limiter l'enfoncement du corps de la seringue lorsqu'il arrive dans sa deuxième position, de telle manière que l'extrémité des moyens de mise en communication (34, 60) dans cette position soit sensiblement dans le plan de la face interne dudit bouchon (22).

21. Dispositif de seringue selon la revendication 20, caractérisé en ce que lesdits moyens pour limiter l'enfoncement comprennent un épaulement faisant partie intégrante desdits premiers moyens d'étanchéité.

22. Dispositif de seringue selon la revendication 5, caractérisé en ce que après rupture desdits moyens de solidarisation (28) et enlèvement du flacon (10), ladite première extrémité de moyens de guidage forme une zone d'appui pour l'injection et en ce que lesdits moyens de guidage comprennent un épaulement interne (85) pour limiter l'enfoncement de la seringue dans lesdits moyens de guidage (14) au-delà de ladite zone d'appui de façon à ce que l'extrémité de l'aiguille fasse saillie hors de ladite zone d'appui.

23. Dispositif de seringue selon la revendication 1, caractérisé en ce que ledit flacon (10') comporte à son extrémité opposée à ladite ouverture (18) une membrane perforable (94).

24. Dispositif de seringue selon la revendication 23, caractérisé en ce que ledit bouchon (102) est monté coulissant de façon étanche à l'intérieur dudit flacon (10') et comporte des moyens de solidarisation temporaire audit flacon (10') à proximité de son ouverture (18).

25. Dispositif de seringue selon la revendication 24, caractérisé en ce que lesdits moyens de solidarisation temporaire comprennent un premier filetage (106) ménagé dans ledit bouchon (102) apte à coopérer avec un deuxième filetage (114) ménagé à l'extrémité (108) de raccordement de ladite pièce de guidage (14').

26. Dispositif de seringue selon l'une quelconque des revendications 1 à 25, caractérisé en ce que ledit bouchon (22, 102) est préperforé.

27. Dispositif de seringue selon l'une quelconque des revendications 9 et 12 à 16, caractérisé en ce que les moyens de guidage (14, 14') comportent extérieurement une portion en saillie (51, 154) apte à protéger les doigts par rapport à l'aiguille lorsqu'on tient les moyens de guidage.

28. Dispositif de seringue selon la revendication 1, caractérisé en ce que ledit joint a la forme d'une bague dont la face coopérant avec le corps de la seringue comporte au moins deux lèvres d'étanchéité.

29. Dispositif de seringue selon l'une des revendications 1 et 28, caractérisé en ce qu'il comprend en outre des moyens annulaires de protection entourant une partie du corps de ladite seringue pour interdire l'accès manuel à la face externe de ladite partie du corps de seringue, une première extrémité desdits moyens de protection étant adjacente auxdits premiers moyens d'étanchéité lorsque ladite seringue occupe sa première position dans lesdits moyens de guidage, la longueur axiale l des moyens de protection entre sa première et sa deuxième extrémité étant au moins égale à la course l' de ladite seringue dans lesdits moyens de guidage entre sa première et sa deuxième position.

30. Dispositif de seringue selon la revendication 29, caractérisé en ce que lesdits moyens annulaires de protection comprennent une jupe cylindrique prolongeant l'extrémité externe des premiers moyens d'étanchéité, ladite jupe présentant un diamètre interne supérieur au diamètre externe du corps de ladite seringue.

31. Dispositif de seringue selon la revendication 30, caractérisé en ce que lesdits moyens annulaire de protection comprennent un manchon monté glissant sur le corps de ladite seringue, ledit manchon comportant une première extrémité qui, lorsque la seringue est dans sa première position, est adjacente à l'extrémité externe desdits premiers moyens d'étanchéité.

32. Dispositif de seringue selon l'une quelconque des revendications 1 à 31, caractérisé en ce que ledit joint est en matériau élastomérique.

33. Dispositif de seringue selon l'une quelconque des revendications 1 à 32, caractérisé en ce que ledit joint est en matériau plastique.

34. Dispositif de seringue selon la revendication 1, caractérisé en ce que ledit joint comprend des moyens temporaires de solidarisation en translation avec le corps de ladite seringue lorsque ladite seringue est déplacée entre sa première et sa deuxième position.

35. Dispositif de seringue selon la revendication 34, caractérisé en ce que les moyens de solidarisation temporaires comprennent un épaulement ménagé dans la face du joint coopérant avec un relief du corps de la seringue, par quoi ledit joint est entraîné lors de l'enfoncement du corps de seringue.

36. Dispositif de seringue selon l'une quelconque des revendications 34 et 35, caractérisé en ce que lesdits moyens de coopération assurent une étanchéité entre ledit joint et ledit corps de seringue.

37. Dispositif de seringue selon l'une quelconque des revendications 34 à 36, caractérisé en ce que ledit joint comporte un premier élément de verrouillage apte à coopérer avec un deuxième élément de verrouillage lorsque ledit joint est dans sa deuxième position.

38. Dispositif de seringue selon l'une quelconque des revendications 34 à 37, caractérisé en ce que la face dudit joint coopérant avec la pièce de guidage comporte au moins deux lèvres d'étanchéité.

39. Dispositif de seringue selon l'une quelconque des revendications 1 et 34 à 38, caractérisé en ce que les premiers moyens d'étanchéité (274) consistent en un joint en forme de bague annulaire solidaire de la seringue en translation dans le sens de l'enfoncement, ladite bague présentant sur sa face externe des lèvres d'étanchéité (278, 280, 282) coopérant avec la face interne des moyens de guidage (14''''), et en ce que ladite pièce de guidage présente dans sa face interne des languettes escamotables (284,286) aptes à coopérer avec lesdites lèvres pour empêcher la sortie dudit joint hors de la pièce de guidage.

40. Dispositif de seringue selon l'une quelconque des revendications 1 à 27, caractérisé en ce que lesdits premiers moyens d'étanchéité consistent en une bague annulaire (312) solidaire de la seringue en translation dans le sens de l'enfoncement, ladite bague présentant sur ses faces internes et externes des lèvres d'étanchéité et en ce que ladite pièce de guidage (14'''') présente un épaulement (314) pour empêcher le sortie dudit joint hors de ladite pièce.

41. Dispositif de seringue selon la revendication 39, caractérisé en ce que ladite bague comprend trois lèvres d'étanchéité et en ce que, dans la position de stockage, lesdites languettes sont disposées entre la lèvre la plus extérieure (282) et la lèvre intermédiaire (280).

42. Dispositif de seringue selon l'une quelconque des revendications 1 à 41, caractérisé en ce que les moyens de guidage comportent en outre des deuxièmes moyens d'étanchéité aptes à assurer avec les premiers moyens d'étanchéité un confinement des moyens de mise en communication à l'intérieur desdits moyens de guidage.

43. Dispositif de seringue selon la revendication 42, caractérisé en ce que lesdits deuxièmes moyens d'étanchéité recouvrent la portion du bouchon (22) dudit flacon destinée à être perforée par les moyens de mise en communication.

44. Dispositif de seringue selon la revendication 43, caractérisé en ce que ladite première extrémité des moyens de guidage comporte une collerette apte à prendre appui sur l'ouverture dudit flacon, et des moyens de fixation sur ledit flacon, ladite collerette comportant dans sa face tournée vers le flacon une portion annulaire en saillie pour coopérer de façon étanche avec le bouchon dudit flacon.

45. Dispositif de seringue selon l'une quelconque des revendications 1 à 27, caractérisé en ce que lesdits premiers moyens d'étanchéité (250, 252) font partie intégrante du corps de la seringue.

46. Dispositif de seringue selon la revendication 45, caractérisé en ce que le corps de la seringue est réalisé en un matériau plastique et en ce que lesdits premiers moyens d'étanchéité consistent en au moins une lèvre annulaire faisant saillie du corps de la seringue et coopérant avec la face interne des moyens de guidage.

47. Dispositif de seringue selon la revendication 45, caractérisé en ce que la seringue est constituée par une carpule (236) prolongée par un embout (246) en matériau plastique à l'extrémité duquel est montée une aiguille (34'), ledit embout présentant sur sa face externe au moins une nervure annulaire (250, 252) coopérant avec la face interne des moyens de guidage (14a), ladite nervure constituant lesdits premiers moyens d'étanchéité.

48. Dispositif de seringue selon la revendication 47, caractérisé en ce que ladite agiuille (34') est vissée sur ledit embout (246), la tête (248) de ladite aiguille étant munie de nervures rayonnantes (254) aptes à coopérer avec une portion (260) formant saillie à l'intérieur de la pièce de guidage (14a), par quoi la rotation du corps de seringue (12') permet le dévissage de ladite aiguille (34') par rapport à l'embout (246).

49. Dispositif de seringue selon l'une quelconque des revendications 1 à 48, caractérisé en ce que lesdits moyens de guidage (14, 14', 14'', 14''', 14'''') comprennent intérieurement une membrane élastique, étanche et perforable (270) disposée entre les première et deuxième extrémités desdits moyens de guidage ladite membrane étant traversable, lorsque le dispositif est dans sa position de stockage, par lesdits moyens de mise en communication, la zone entre ladite membrane (270) et lesdits bouchons formant une chambre de confinement permettant de capter les gaz susceptibles de sortir dudit flacon.

50. Dispositif de seringue selon l'une quelconque des revendications 1 à 49, caractérisé en ce que lesdits moyens de guidage comportent en outre entre leurs première et deuxième extrémités un orifice obturé par un filtre micro poreux.

51. Dispositif de seringue selon l'une quelconque des revendications 1 à 48, caractérisé en ce que ladite pièce de guidage (14'''') comporte à proximité de sa première extrémité une pièce (300) en matériau poreux, traversé par ladite aiguille (34) apte à absorber les éventuels gaz sortant dudit flacon lors de l'extraction de l'aiguille hors de celui-ci.

52. Dispositif de seringue selon l'une quelconque des revendications 1 à 50, caractérisé en ce que ladite première extrémité de la pièce de guidage (14'''') est prolongée par un logement (302) raccordé à l'intérieur de la pièce de guidage par un passage (304) pour le passage de l'aiguille (34), le bord libre (306) dudit logement étant appliqué contre le bouchon (22) dudit flacon, de telle manière que ledit logement constitue une enceinte de confinement pour les gaz susceptibles de sortir dudit flacon.

53. Dispositif de seringue selon la revendication 9, caractérisé en ce qu'il comporte en outre un protège-aiguille (180) monté de façon amovible autour de ladite aiguille, ledit protège-aiguille comportant une extrémité perforante s'étendant au-delà de la pointe de ladite aiguille, ladite extrémité perforante étant apte à perforer le bouchon dudit flacon.

54. Dispositif de seringue selon la revendication 53, caractérisé en ce que ledit protège-aiguille (180) comprend à son extrémité de fixation (186) une portion (187) en matériau élastique coopérant de façon étanche avec l'extrémité de ladite seringue (12) sur laquelle est montée l'aiguille (34).

55. Dispositif de seringue selon l'une quelconque des revendications 1 à 54, caractérisé en ce qu'il comprend en outre une tige de commande d'un piston de la seringue, ladite tige étant vissable dans ledit piston (52).

56. Dispositif de seringue selon l'une quelconque des revendications 1 à 55, caractérisé en ce qu'il comprend en outre un dispositif d'auto-injection montable sur l'ensemble constitué pr ladite seringue (12) et lesdits moyens de guidage (14, 14', 14'', 14''') après séparation dudit flacon (10), ledit dispositif d'auto-injection comprenant un support (202) des moyens de guidage sur ledit support, des premiers moyens poussoirs (212) pour provoquer l'enfoncement du corps de la seringue (12) par rapport auxdits moyens de guidage (14''') d'une première distance prédéterminée et des deuxièmes moyens poussoirs (216) pour provoquer l'enfoncement dudit piston (52) par rapport au corps de ladite seringue d'une deuxième distance prédéterminée, sans modifier la position relative de la seringue et des moyens de guidage, ladite première extrémité des moyens de guidage formant une zone d'appui sur le corps du patient.

57. Dispositif de seringue selon l'une quelconque des revendications 1 à 56, caractérisé en ce qu'il comprend des moyens de contrôle de sortie de la seringue hors desdits moyens de guidage comportant des moyens pour n'autoriser la sortie de la seringue hors des moyens de guidage que partiellement de telle manière que les moyens de mise en communication restent dans lesdits moyens de guidage, et des moyens pour n'autoriser la sortie complète de la seringue qu'après avoir imprimé à la seringue un mouvement de rotation par rapport auxdits moyens de guidage.

58. Dispositif de seringue selon la revendication 57, caractérisé en ce que lesdits moyens de contrôle comprennent un ergot (322) faisant saillie hors de la seringue et une rainure (324), non parallèle à la direction de sortie de la seringue, ledit ergot étant engagé dans ladite rainure lorsque ladite seringue est dans lesdits moyens de guidage.

59. Dispositif de seringue selon la revendication 58, caractérisé en ce que ladite rainure est ménagée dans la face interne desdits premiers moyens d'étanchéité.

## Claims

1. Syringe device making it possible to mix two compounds, at least the first of which is liquid, comprising:
a syringe (12) having a cylindrical body provided with an injection end (32), the syringe containing said first compound, further comprising:
means (14, 14', 14'', 14''', 14'''') for guiding the injection end of said syringe in translation between a first withdrawn so-called storage position and a second inserted so-called active position, said guide means including an internal face, a first end intended to emerge opposite the perforable zone of a stopper capable of closing in leaktight manner the opening of a vial intended to contain one of said compounds and a second end (46, 204) for receiving the injection end of said syringe, said guide means being leaktight between their first and second ends, said second end including sealing means and means for providing sufficient linkage between said syringe and said guide means while allowing said translation; and
communication means (34, 60) arranged, along the axis of the body of said syringe, so as to allow perforation of said perforable zone of said stopper and communication of the internal volume of said syringe with the inside of said vial when the injection end of said syringe is brought into its second position,
said device being characterized in that said sealing means include a seal (48, 158, 206, 246, 274) mounted inside said guide means, said seal being capable of providing sealing between the body of the syringe and the internal face of the guide means in the storage position and during the relative displacements of the two pieces.

2. Syringe device according to claim 1, characterized in that it comprises said vial provided with the stopper and in that it further comprises means (28, 108, 222, 222') for linking said first end of the guide means on the opening (18) of said vial (10,10').

3. Syringe device according to claim 2, characterized in that said vial (10) is provided with a bottom.

4. Syringe device according to claim 3, characterized in that the means (28) for linking the guide piece (14) on said vial furthermore comprise means for leaktight attachment of the periphery of said stopper (22) on said vial (10).

5. Syringe device according to any one of claims 1 to 4, characterized in that said linkage means (28, 222, 222') can be broken in order to allow detachment of the vial (10) with respect to the guide piece (14, 14', 14'', 14''').

6. Syringe device according to any one of claims 3 to 5, characterized in that said linkage means comprise a crimping capsule (222, 222') mounted on said first end (220, 220') of said guide means (14''') and capable of being crimped into a collar (20) of the opening of said vial (10) during the fastening of said guide means on said vial.

7. Syringe device according to any one of claims 3 to 5, characterized in that said fastening means comprise clip tabs interacting with the periphery of the opening of the vial.

8. Syringe device according to claim 7, characterized in that said clip means furthermore comprise a crimping ring interacting with the clip tabs.

9. Syringe device according to any one of claims 1 to 8, characterized in that the communication means comprise a needle (34) mounted at the injection end of the syringe and whose tip is capable of perforating the central zone of the stopper (22) when the end of the syringe is brought into its second position.

10. Syringe device according to any one of claims 1 to 9, characterized in that the communication means comprise a tubular piece (60), a first end (64) of which is capable of closing the central zone of the stopper (22) and the other end (62) of which is capable of piercing a perforable partition (70) made at the injection end (32) of the syringe (12) when the latter is brought into its second position.

11. Syringe device according to claim 10, characterized in that said tubular piece consists of two needles between which a filter is arranged.

12. Syringe device according to claim 9, characterized in that it furthermore comprises a cap (36) surrounding said needle (34) and one end of which is pierceable by the tip of said needle when the end of said syringe is brought into its second position.

13. Syringe device according to claim 12, characterized in that said cap (36) is made of a deformable elastic material and in that it extends over the entire length of the needle when the injection end (32) of the syringe is in its first position.

14. Syringe device according to any one of claims 12 and 13, characterized in that said cap (36) comprises translational immobilization means (42) inside said guide means (14).

15. Syringe device according to claim 9, characterized in that it furthermore comprises a perforable piece (80, 120) integral with the guide means (14) and into which the end of the needle (34) is inserted.

16. Syringe device according to claim 15, characterized in that said perforable piece (80, 120) is preperforated.

17. Syringe device according to any one of claims 15 and 16, characterized in that said perforable piece is made of an elastic material and in that the face of said piece intended to be applied against the stopper of said vial is arranged, with respect to said guide means, such that when said guide piece is fixed on said vial, said perforable piece is compressed.

18. Syringe device according to any one of claims 9, 12 to 15, characterized in that it furthermore comprises an axial guide tube (82) integral with said guide means (14) and surrounding the needle when the syringe is fitted into the guide means.

19. Syringe device according to any one of claims 1 to 18, characterized in that said guide means (14) have a first internal shoulder (49) for limiting the insertion of the body of the syringe into said guide means, such that the end of the needle does not perforate the stopper (22) in the storage situation.

20. Syringe device according to any one of claims 1 to 19, characterized in that said guide means comprise means (50) for limiting the insertion of the body of the syringe when it arrives in its second position, such that the end of the communication means (34, 60) in this position is substantially in the plane of the internal face of said stopper (22).

21. Syringe device according to claim 20, characterized in that said means for limiting the insertion comprise a shoulder forming an integral part of said first sealing means.

22. Syringe device according to claim 5, characterized in that, after breakage of said linkage means (28) and removal of the vial (10), said first end of guide means forms a bearing zone for the injection and in that said guide means comprise an internal shoulder (85) for limiting the insertion of the syringe into said guide means (14) beyond said bearing zone so that the end of the needle projects out of said bearing zone.

23. Syringe device according to claim 1, characterized in that said vial (10') comprises, at its end opposite said opening (18), a perforable membrane (94).

24. Syringe device according to claim 23, characterized in that said stopper (102) is mounted sliding in leaktight manner inside said vial (10') and includes means for temporary linkage to said vial (10') in proximity to its opening (18).

25. Syringe device according to claim 24, characterized in that said temporary linkage means comprise a first screw thread (106) made in said stopper (102) and capable of interacting with a second screw thread (114) made at the connection end (108) of said guide piece (14').

26. Syringe device according to any one of claims 1 to 25, characterized in that said stopper (22, 102) is preperforated.

27. Syringe device according to any one of claims 9 and 12 to 16, characterized in that the guide means (14, 14') externally include a projecting portion (51, 154) capable of protecting the fingers with respect to the needle when the guide means are held.

28. Syringe device according to claim 1, characterized in that said seal has the form of a ring whose face which interacts with the body of the syringe includes at least two sealing lips.

29. Syringe device according to one of claims 1 and 28, characterized in that it furthermore comprises annular protection means surrounding a part of the body of said syringe in order to prevent manual access to the external face of said part of the syringe body, a first end of said protection means being adjacent to said first sealing means when said syringe occupies its first position in said guide means, the axial length l of the protection means between its first end and its second end being at least equal to the travel l' of said syringe in said guide means between its first position and its second position.

30. Syringe device according to claim 29, characterized in that said annular protection means comprise a cylindrical skirt extending the external end of the first sealing means, said skirt having an internal diameter greater than the external diameter of the body of said syringe.

31. Syringe device according to claim 30, characterized in that said annular protection means comprise a sleeve mounted slidingly on the body of said syringe, said sleeve including a first end which, when the syringe is in its first position, is adjacent to the external end of said first sealing means.

32. Syringe device according to any one of claims 1 to 31, characterized in that said seal is made of elastomer.

33. Syringe device according to any one of claims 1 to 32, characterized in that said seal is made of plastic.

34. Syringe device according to claim 1, characterized in that said seal comprises temporary means for translational linkage with the body of said syringe when said syringe is displaced between its first position and its second position.

35. Syringe device according to claim 34, characterized in that said temporary linkage means comprise a shoulder made on the face of the seal interacting with a relief of the body of the syringe, by which said seal is moved along when the syringe body is inserted.

36. Syringe device according to any one of claims 34 and 35, characterized in that said interaction means provide sealing between said seal and said syringe body.

37. Syringe device according to any one of claims 34 to 36, characterized in that said seal includes a first locking element capable of interacting with a second locking element when said seal is in its second position.

38. Syringe device according to any one of claims 34 to 37, characterized in that the face of said seal interacting with the guide piece includes at least two sealing lips.

39. Syringe device according to any one of claims 1 and 34 to 38, characterized in that the first sealing means (274) consist of a seal having the shape of an annular ring linked in translation with the syringe in the insertion direction, said ring having, on its external face, sealing lips (278, 280, 282) interacting with the internal face of the guide means (14'''), and in that said guide piece has, in its internal face, retractable tongues (284, 286) capable of interacting with said lips, whereby said seal is prevented from leaving said guide piece.

40. Syringe device according to any one of claims 1 to 27, characterized in that said first sealing means consist of an annular ring (312) linked in translation with the syringe in the insertion direction, said ring having, on its internal and external faces, sealing lips, and in that said guide piece (14'''') includes a shoulder (314) to prevent said seal from leaving said guide piece.

41. Syringe device according to claim 39, characterized in that said ring comprises three sealing lips and in that, in the storage position, said tongues are arranged between the outermost lip (282) and the intermediate lip (280).

42. Syringe device according to any one of claims 1 to 41, characterized in that the guide means furthermore include second sealing means capable of providing, with the first sealing means, confinement of the communication means inside said guide means.

43. Syringe device according to claim 42, characterized in that said second sealing means cover the portion of the stopper (22) of said vial intended to be perforated by the communication means.

44. Syringe device according to claim 43, characterized in that said first end of the guide means includes a collar capable of bearing on the opening of said vial, and means of fastening on said vial, said collar including, in its face turned towards the vial, a projecting annular portion for interacting in leaktight manner with the stopper of said vial.

45. Syringe device according to any one of claims 1 to 27, characterized in that said first sealing means (250, 252) form an integral part of the syringe body.

46. Syringe device according to claim 45, characterized in that the syringe body is made of plastic and in that said first sealing means consist of at least one annular lip projecting from the syringe body and interacting with the internal face of the guide means.

47. Syringe device according to claim 45, characterized in that the syringe consists of a carpule (236) extended by a plastic adapter (246), at the end of which a needle (34') is mounted, said adapter having on its external face at least one annular rib (250, 252) interacting with the internal face of the guide means (14a), said rib constituting said first sealing means.

48. Syringe device according to claim 47, characterized in that said needle (34') is screwed onto said adapter (246), the head (248) of said needle being provided with radial ribs (254) capable of cooperating with a portion (260) forming a projection into the guide piece (14a), by which rotation of the syringe body (12') allows unscrewing of said needle (34') with respect to the adapter (246).

49. Syringe device according to any one of claims 1 to 48, characterized in that said guide means (14, 14', 14'', 14''', 14'''') internally comprise a perforable, elastic, leaktight membrane (270) arranged between the first end and the second end of said guide means, said membrane being passed through, when the device is in its storage position, by said communication means, whereby the zone located between said membrane (270) and said stoppers forms a retention chamber making it possible to collect the gases which may leave said vial.

50. Syringe device according to any one of claims 1 to 49, characterized in that said guide means furthermore include, between their first end and their second end, an orifice closed by a micropore filter.

51. Syringe device according to any one of claims 1 to 48, characterized in that said guide piece (14'''') includes, in proximity to its first end, a piece (300) made of porous material, passed through by said needle (34) and capable of absorbing possible gases leaving said vial during the extraction of the needle therefrom.

52. Syringe device according to any one of claims 1 to 50, characterized in that said first end of the guide piece (14'''') is extended by a housing (302) connected to the inside of the guide piece by a passage (304) for passage of the needle (34), the free edge (306) of said housing being applied against the stopper (22) of said vial, whereby said housing forms a retention chamber for the gases which may leave said vial.

53. Syringe device according to claim 9, characterized in that it furthermore includes a needle protector (180) removably mounted around said needle, said needle protector including a perforating end extending beyond the tip of said needle, said perforating end being capable of perforating the stopper of said vial.

54. Syringe device according to claim 53, characterized in that said needle protector (180) comprises, at its fastening end (186), a portion (187) made of elastic material, interacting in leaktight manner with the end of said syringe (12) on which the needle (34) is mounted.

55. Syringe device according to any one of claims 1 to 54, characterized in that it furthermore comprises a control rod for a plunger of the syringe, said rod being screwable into said plunger (52).

56. Syringe device according to any one of claims 1 to 55, characterized in that it furthermore comprises an automatic injection device mountable on the assembly consisting of said syringe (12) and said guide means (14, 14', 14'', 14''') after separation of said vial (10), said automatic injection device comprising a support (202) for the guide means on said support, first pusher means (212) for causing insertion of the body of the syringe (12) with respect to said guide means (14''') by a first predetermined distance and second pusher means (216) for causing insertion of said plunger (52) with respect to the body of said syringe by a second predetermined distance, without altering the relative position of the syringe and of the guide means, said first end of the guide means forming a zone for bearing on the body of the patient.

57. Syringe device according to any one of claims 1 to 56, characterized in that it comprises exit means for controlling the syringe outside said guide means comprising means for allowing the exit of the syringe from the guide means only in part such that the communication means remains in said guide means, and means allowing the complete exit of the syringe only once a rotation movement has been imparted to the syringe with respect to said guide means.

58. Syringe device according to claim 57, characterized in that said control means comprise a snug (322) projecting outside the syringe and a notch (324), which is not parallel to the exit direction of the syringe, said snug being engaged in said notch when said syringe is inside said guide means.

59. Syringe device according to claim 58, characterized in that said notch is provided inside the internal face of said first sealing means.

## Patentansprüche

1. Spritzenvorrichtung zur Vermischung Zweier Verbindungen, von denen mindestens die erste flüssig ist, umfassend:
eine Spritze (12), die einen Zylinderkörper aufweist, der mit einem Injektionsende (32) versehen ist, wobei die Spritze die genannte erste Verbindung enthält, und wobei ausserdem enthalten sind:
Elemente (14, 14', 14'', 14''', 14'''') zur in Translation gerichteter Führung des Injektionsendes der genannten Spritze zwischen einer ersten Rückzugsstellung als sogenannter Lagerstellung und einer zweiten hineingedrückten Stellung als sogenannter Aktivstellung, wobei die genannten Führungselemente eine Innenfläche, ein erstes Ende, bestimmt zur Mündung in Richtung auf die perforierbare Zone eines Stopfens, der geeignet ist, die Öffnung eines Fläschchens dicht zu verschließen, das dazu bestimmt ist, eine der beiden Verbindungen zu enthalten, und ein zweites Ende (46, 204) zur Aufnahme des Injektionsendes der genannten Spritze umfassen, und wobei die genannten Führungselemente zwischen ihrem ersten und zweiten Ende dicht sind, wobei das genannte zweite Ende Dichtungselemente und Elemente umfaßt, um eine ausreichende Verbindung zwischen der genannten Spritze und den genannten Führungselementen sicherzustellen, wobei insgesamt die genannte Translation gewährleistet ist; sowie
Elemente zur Bereitstellung von Verbindungswegen (34, 60), die entlang der Achse des Körpers der genannten Spritze angeordnet sind, um die Perforation der genannten perforierbaren Zone des genannten Stopfens und die Bereitstellung eines Verbindungswegs des Innenvolumen der genannten Spritze zum Inneren des genannten Fläschchens zu ermöglichen, wenn das Injektionsende der genannten Spritze in seine zweite Stellung geführt wird,
wobei die Vorrichtung dadurch gekennzeichnet ist, daß die genannten Dichtungselemente eine Dichtung (48, 158, 206, 246, 274) einschließen, die im Inneren der genannten Führungselemente angebracht ist, wobei die genannte Dichtung geeignet ist, eine Dichtheit zwischen dem Spritzenkörper und der Innenfläche der Führungselemente in Lagerstellung und bei Relativverschiebungen der beiden Stücke sicherzustellen.

2. Spritzenvorrichtung nach Anspruch 1,
dadurch **gekennzeichnet**, daß
sie das genannte Fläschchen, das mit einem Stopfen versehen ist, und ausserdem Verbindungselemente (28, 108, 222, 222') des genannten ersten Endes der Führungselemente auf der Öffnung (18) des genannten Fläschchens (10, 10') umfaßt.

3. Spritzenvorrichtung gemäß Anspruch 2,
dadurch **gekennzeichnet**, daß
das genannte Fläschchen (10) mit einem Boden versehen ist.

4. Spritzenvorrichtung gemäß Anspruch 3,
dadurch **gekennzeichnet**, daß
die Verbindungselemente (28) des Führungsstücks (14) auf dem genannten Fläschchen ausserdem Elemente zur dichten Verbindung des Aussenbereichs des genannten Stopfens (22) auf dem genannten Fläschchen (10) umfassen.

5. Spritzenvorrichtung gemäß einem der Ansprüche 1 bis 4,
dadurch **gekennzeichnet**, daß
die genannten Verbindungselemente (28, 222, 222') gelöst werden können, um die Ablösung des Fläschchens (10) bezüglich dem Führungsstück (14, 14', 14'', 14''') zu ermöglichen.

6. Spritzenvorrichtung gemäß einem der Ansprüche 3 bis 5,
dadurch **gekennzeichnet**, daß
die genannten Verbindungselemente eine Fassungskapsel (222, 222') umfassen, die auf dem genannten ersten Ende (220, 220') der genannten Führungselemente (14''') montiert sind und sich dazu eignen, auf einem Krägelchen (20) der Öffnung des genannten Fläschchens (10) bei der genannten Fixierung der genannten Führungselemente auf dem genannten Fläschchen eingefügt zu werden.

7. Spritzenvorrichtung gemäß einem der Ansprüche 3 bis 5,
dadurch **gekennzeichnet**, daß
die genannten Führungselemente Klammerhalterungen umfassen, die mit dem Aussenbereich der Öffnung des Fläschchens verbunden sind.

8. Spritzenvorrichtung gemäß Anspruch 7,
dadurch **gekennzeichnet**, daß
die genannten Klammerelemente ausserdem einen Fassungsring umfassen, der mit den Klammerhalterungen verbunden ist.

9. Spritzenvorrichtung gemäß einem der Ansprüche 1 bis 8,
dadurch **gekennzeichnet**, daß
die Elemente zur Bereitstellung von Verbindungswegen eine Nadel (34) umfassen, die am Injektionsende der Spritze montiert ist und deren Spitze sich dazu eignet, die Zentralzone des Stopfens (22) zu perforieren, wenn das Ende der Spritze in ihre zweite Stellung geführt wird.

10. Spritzenvorrichtung gemäß einem der Ansprüche 2 bis 9,
dadurch **gekennzeichnet**, daß
die Elemente zur Bereitstellung von Verbindungswegen ein rohrförmiges Stück (60) umfassen, dessen erstes Ende (64) geeignet ist, die Zentralzone des Stopfens (22) zu verschließen, und dessen anderes Ende (62) dazu geeignet ist, eine perforierbare Trennwand (70) zu durchstoßen, die am Injektionsende (32) der Spritze (12) vorgesehen ist, wenn diese in ihre zweite Stellung geführt wird.

11. Spritzenvorrichtung gemäß Anspruch 10,
dadurch **gekennzeichnet**, daß
das genannte rohrförmige Stück aus zwei Nadeln zusammengesetzt ist, zwischen denen ein Filter angeordnet ist.

12. Spritzenvorrichtung gemäß Anspruch 9,
dadurch **gekennzeichnet**, daß
sie ausserdem eine Kappe (36) umfaßt, die die genannte Nadel (34) umgibt und deren eines Ende von der Spitze der genannten Nadel durchstechbar ist, wenn das Ende der genannten Spritze in seine zweite Stellung geführt wird.

13. Spritzenvorrichtung gemäß Anspruch 12,
dadurch **gekennzeichnet**, daß
die genannte Kappe (36) aus einem elastischen deformierbaren Material hergestellt ist und sich über die ganze Länge der Nadel erstreckt, wenn das Injektionsende (32) der Spritze in seiner ersten Stellung vorliegt.

14. Spritzenvorrichtung gemäß einem der Ansprüche 12 und 13,
dadurch **gekennzeichnet**, daß
die genannte Kappe (36) Elemente (42) zur Immobilisierung der Translationsrichtung im Inneren der genannten Führungselemente (14) umfaßt.

15. Spritzenvorrichtung gemäß Anspruch 9,
dadurch **gekennzeichnet**, daß
sie ausserdem ein perforierbares Stück (80, 120) umfaßt, das mit den Führungselementen (14) verbunden ist und in das das Ende der Nadel (34) hineingedrückt wird.

16. Spritzenvorrichtung gemäß Anspruch 15,
dadurch **gekennzeichnet**, daß
das genannte perforierbare Stück (80, 120) vorperforiert ist.

17. Spritzenvorrichtung gemäß einem der Ansprüche 15 und 16,
dadurch **gekennzeichnet**, daß
das genannte perforierbare Stück aus einem elastischen Material hergestellt und die zur Anwendung gegen den Stopfen des genannten Fläschchens bestimmte Seite des genannten Stücks bezüglich den genannten Führungselementen so angeordnet sind, daß, bei Fixierung des genannten Führungsstücks auf dem genannten Fläschchen, das genannte perforierbare Stück zusammengedrückt wird.

18. Spritzenvorrichtung gemäß einem der Ansprüche 9 und 12 bis 15,
dadurch **gekennzeichnet**, daß
sie ausserdem ein axiales Führungsrohr (82) umfaßt, das mit den genannten Führungselementen (14) verbunden ist und die Nadel umgibt, wenn die Spritze in die Führungselemente eingeführt wird.

19. Spritzenvorrichtung gemäß einem der Ansprüche 1 bis 18,
dadurch **gekennzeichnet**, daß
die genannten Führungselemente (14) eine erste Innenschulter (49) aufweisen, um das Hineindrücken des Spritzenkörpers in die genannten Führungselemente zu begrenzen, und zwar so, daß das Ende der Nadel den Stopfen (22) in Lagerstellung nicht perforiert.

20. Spritzenvorrichtung gemäß einem der Ansprüche 1 bis 19,
dadurch **gekennzeichnet**, daß
die genannten Führungselemente Elemente (50) umfassen, um das Hineindrücken des Spritzenkörpers zu begrenzen, wenn er an seiner zweiten Stellung ankommt, und zwar so, daß das Ende der Verbindungswege (34, 60) in dieser Stellung genau in der Ebene der Innenfläche des genannten Stopfens (22) vorliegt.

21. Spritzenvorrichtung gemäß Anspruch 20,
dadurch **gekennzeichnet**, daß
die genannten Elemente zur Begrenzung des Hineindrückens eine Schulter umfassen, die einen integralten Teil der genannten ersten Dichtungselemente bildet.

22. Spritzenvorrichtung gemäß Anspruch 5,
dadurch **gekennzeichnet**, daß
nach Ablösung der genannten Verbindungselemente (28) und Entfernung des Fläschchens (10) das genannte erste Ende der Führungselemente eine Druckzone für die Injektion bildet und die genannten Führungselemente eine Innenschulter (85) zur Begrenzung des Hineindrückens der Spritze in die genannten Führungselemente (14) hin zu/weg von der genannten Druckzone umfassen, und zwar so, daß das Ende der Nadel von der genannten Druckzone wegragt.

23. Spritzenvorrichtung gemäß Anspruch 1,
dadurch **gekennzeichnet**, daß
das genannte Fläschchen (10') an seinem der genannten Öffnung (18) gegenüberliegenden Ende eine perforierbare Membran (94) aufweist.

24. Spritzenvorrichtung gemäß Anspruch 23,
dadurch **gekennzeichnet**, daß
der genannte Stopfen (102) gleitend in dichter Weise gegenüber dem Inneren des genannten Fläschchen (10') angebracht ist und Elemente zur zeitweisen Verbindung mit dem genannten Fläschchen (10') in der Nähe seiner Öffnung (18) aufweist.

25. Spritzenvorrichtung gemäß Anspruch 24,
dadurch **gekennzeichnet**, daß
die genannten Elemente zur zeitweisen Verbindung ein erstes Gewinde (106) umfassen, das in dem genannten Stopfen (102) vorgesehen ist und sich dazu eignet, mit einem zweiten Gewinde (114) zusammenzuwirken, das am Ende (108) der Verbindung des genannten Führungsstücks (14') vorgesehen ist.

26. Spritzenvorrichtung gemäß einem der Ansprüche 1 bis 25,
dadurch **gekennzeichnet**, daß
der genannte Stopfen (22, 102) perforiert ist.

27. Spritzenvorrichtung gemäß einem der Ansprüche 9 und 12 bis 16,
dadurch **gekennzeichnet**, daß
die Führungselemente (14, 14') aussen einen Vorsprungteilbereich (51, 154) aufweisen, der sich dazu eignet, die Finger bezüglich der Nadel zu schützen, wenn man die Führungselemente hält.

28. Spritzenvorrichtung gemäß Anspruch 1,
dadurch **gekennzeichnet**, daß
die genannte Dichtung die Form eines Rings aufweist, dessen mit dem Spritzenkörper verbundene Seite mindestens zwei Dichtungslippen aufweist.

29. Spritzenvorrichtung gemäß einem der Ansprüche 1 bis 28,
dadurch **gekennzeichnet**, daß
sie ausserdem ringförmige Schutzelemente enthält, die einen Teil des Körpers der genannten Spritze umgeben, um einen händischen Zugriff auf die Aussenfläche des genannten Teilbereichs des genannten Spritzenkörpers zu unterbinden, wobei ein erstes Ende der genannten Schutzelemente an die genannten ersten Dichtungselemente angrenzt, wenn die genannte Spritze ihre erste Stellung in den genannten Führungselementen einnimmt, wobei die Axiallänge L der Schutzelemente zwischen ihrem ersten und zweiten Ende mindestens gleich dem Verlauf der genannten Spritze in den genannten Führungselementen zwischen ihrer ersten und zweiten Stellung ist.

30. Spritzenvorrichtung gemäß Anspruch 29,
dadurch **gekennzeichnet**, daß
die ringförmigen Schutzelemente einen Zylinderfortsatz umfassen, der sich zum Aussenende der ersten Dichtungselemente verlängert, wobei der genannte Fortsatz einen Innendurchmesser aufweist, der größer als der Aussendurchmesser des Körpers der genannten Spritze ist.

31. Spritzenvorrichtung gemäß Anspruch 30.
dadurch **gekennzeichnet**, daß
die genannten ringförmigen Schutzelemente eine Muffe umfassen, die gleitend auf dem Körper der genannten Spritze montiert ist, wobei die genannte Muffe ein erstes Ende umfaßt, das, wenn sich die Spritze in ihrer ersten Stelle befindet, an das Aussenende der genannten Dichtungselemente angrenzt.

32. Spritzenvorrichtung gemäß einem der Ansprüche 1 bis 31,
dadurch **gekennzeichnet**, daß
die genannte Dichtung aus einem elastomeren Material ist.

33. Spritzenvorrichtung gemäß einem der Ansprüche 1 bis 32,
dadurch **gekennzeichnet**, daß
die genannte Dichtung aus einem Kunststoffmaterial ist.

34. Spritzenvorrichtung gemäß Anspruch 1,
dadurch **gekennzeichnet**, daß
die genannte Dichtung zeitlich wirksame Elemente zur Verbindung in Translation mit dem Körper der genannten Spritze umfaßt, wenn die genannte Spritze zwischen ihrer ersten und zweiten Stellung verschoben wird.

35. Spritzenvorrichtung gemäß Anspruch 34,
dadurch **gekennzeichnet**, daß
die zeitweisen Verbindungselemente eine Schulter umfassen, die auf der Seite der Dichtung angebracht ist, die mit einem Relief des Spritzenkörpers verbunden ist, wodurch die genannte Dichtung beim Hineindrücken des Spritzenkörpers eingefaßt ist.

36. Spritzenvorrichtung gemäß einem der Ansprüche 34 und 35,
dadurch **gekennzeichnet**, daß
die genannten Verbindungselemente eine Dichtheit zwischen der genannten Dichtung und dem genannten Spritzenkörper sichern.

37. Spritzenvorrichtung gemäß einem der Ansprüche 34 bis 36,
dadurch **gekennzeichnet**, daß
die genannte Dichtung ein erstes Element zur Verriegelung umfaßt, das sich dazu eignet, mit einem zweiten Verriegelungselement zusammenzuwirken, wenn die genannte Dichtung in ihrer zweiten Stellung vorliegt.

38. Spritzenvorrichtung gemäß einem der Ansprüche 34 bis 37,
dadurch **gekennzeichnet**, daß
die Seite der genannten Dichtung, die mit dem Führungsstück verbunden ist, mindestens zwei Dichtungslippen aufweist.

39. Spritzenvorrichtung gemäß einem der Ansprüche 1 und 34 bis 38,
dadurch **gekennzeichnet**, daß
die ersten Dichtungselemente (274) aus einer Dichtung in Form eines kreisförmigen Rings bestehen, der mit der Spritze in Translation in Richtung des Hineindrückens verbunden ist, wobei der genannte Ring auf seiner Aussenseite Dichtungslippen (278, 280, 282) aufweist, die mit der Innenseite der Führungselemente (14'''') verbunden sind, und daß das genannte Führungsstück auf seiner Innenseite verschiebbare Zungen (284, 286) aufweist, die sich dazu eignen, mit den genannten Lippen zusammenzuwirken, um das Heraustreten der genannten Dichtung aus dem Führungsstück zu verhindern.

40. Spritzenvorrichtung gemäß einem der Ansprüche 1 bis 27,
dadurch **gekennzeichnet**, daß
die genannten ersten Dichtungselemente aus einem kreisförmigen Ring (312) bestehen, der mit der Spritze in Translation in der Richtung des Hineindrückens verbunden ist, wobei der genannte Ring auf seinen Innen- und Aussenseiten Dichtungslippen und das genannte Führungsstück (14'''') eine Schulter (314) aufweisen, um das Heraustreten der genannten Dichtung aus dem genannten Stück zu verhindern.

41. Spritzenvorrichtung gemäß Anspruch 39,
dadurch **gekennzeichnet**, daß
der genannte Ring drei Dichtungslippen aufweist, und, in Lagerstellung, die genannten Zungen zwischen der äußersten Lippe (282) und der Zwischenlippe (280) angeordnet sind.

42. Spritzenvorrichtung gemäß einem der Ansprüche 1 bis 41,
dadurch **gekennzeichnet**, daß
die Führungselemente ausserdem zweite Dichtungselemente umfassen, die sich dazu eignen, mit den ersten Dichtungselementen eine Umgrenzung der Verbindungswege im Inneren der genannten Führungselemente zu sichern.

43. Spritzenvorrichtung gemaß Anspruch 42,
dadurch **gekennzeichnet**, daß
die genannten zweiten Dichtungselemente den Teilbereich des Stopfens (22) des genannten Fläschchens bedecken, der dazu bestimmt ist, von den Verbindungselementen perforiert zu werden.

44. Spritzenvorrichtung gemäß Anspruch 43,
dadurch **gekennzeichnet**, daß
das genannte erste Ende der Führungselemente ein Krägelchen, das geeignet ist, auf die Öffnung des genannten Fläschchens zu drücken, und Fixierelemente auf dem genannten Fläschchen umfaßt, wobei das genannte Krägelchen auf seiner gegen das Fläschchen gedrehten Seite einen ringförmigen Teilbereich als Vorsprung aufweist, um dicht mit dem Stopfen des genannten Fläschchens verbunden zu sein.

45. spritzenvorrichtung gemäß einem der Ansprüche 1 bis 27,
dadurch **gekennzeichnet**, daß
die genannten ersten Dichtungselemente (250, 252) einen integralen Bestandteil des Spritzenkörpers bilden.

46. Spritzenvorrichtung gemäß Anspruch 45,
dadurch **gekennzeichnet**, daß
der Spritzenkörper aus einem Kunststoffmaterial hergestellt ist und die genannten ersten Dichtungselemente aus mindestens einer ringförmigen Lippe bestehen, die aus dem Spritzenkörper herausragt und mit der Innenfläche der Führungselemente verbunden ist.

47. Spritzenvorrichtung nach Anspruch 45,
dadurch **gekennzeichnet**, daß
die Spritze aus einem Abschlußteilstück (236) zusammengsetzt ist, das sich über einen Fortsatz (246) aus Kunststoffmaterial verlängert, an dessen Ende eine Nadel (34') montiert ist, wobei der genannte Fortsatz auf seiner Aussenseite mindestens eine ringförmige Rippe (250, 252) aufweist, die mit der Innenseite der Führungselemente (14a) verbunden ist, wobei die genannte Rippe die genannten ersten Dichtungselemente darstellt.

48. Spritzenvorrichtuna gemäß Anspruch 47,
dadurch **gekennzeichnet**, daß
die genannte Nadel (34') auf dem genannten Fortsatz (246) verschraubt ist, wobei der Kopf (248) der genannten Nadel mit Strahlrippen (254) versehen ist, die sich dazu eignen, mit einem Teilbereich (260) zusammenzuwirken, der in das Innere des Führungsstücks (14a) ragt, wodurch die Drehung des Spritzenkörpers (12') das Abschrauben der genannten Nadel (34') vom Fortsatz (246) ermöglicht.

49. Spritzenvorrichtung gemäß einem der Ansprüche 1 bis 48,
dadurch **gekennzeichnet**, daß
die genannten Führungselemente (14, 14', 14'', 14''', 14'''') innerlich eine elastische, dichte und perforierbare Membran (270) enthalten, die zwischen den ersten und zweiten Enden der genannten Führungselemente angeordnet ist, wobei die genannte Membran, wenn die Vorrichtung in ihrer Lagerstellung vorliegt, von den genannten Verbindungselementen durchquerbar ist, wobei die Zone zwischen der genannten Membran (270) und den genannten Stopfen eine Abgrenzungskammer bilden, welche es ermöglicht, Gase einzufangen, die das genannte Fläschchen verlassen könnten.

50. Spritzenvorrichtung gemäß einem der Ansprüche 1 bis 49,
dadurch **gekennzeichnet**, daß
die genannten Führungselemente ausserdem zwischen ihren ersten und zweiten Enden eine Öffnung enthalten, die mit einem mikroporösen Filter verschlossen ist.

51. Spritzenvorrichtung gemäß einem der Ansprüche 1 bis 48,
dadurch **gekennzeichnet**, daß
das genannte Führungsstück (14'''') in der Nähe seines ersten Endes ein Teilstück (300) aus porösem Material enthält, das von der genannten Nadel (34) durchquert wird und sich dazu eignet, die gegebenenfalls auftretenden Gase zu absorbieren, die aus dem genannten Fläschen beim Herausziehen der Spritze aus diesem entweichen.

52. Spritzenvorrichtung gemäß einem der Ansprüche 1 bis 50,
dadurch **gekennzeichnet**, daß
das genannte erste Ende des Führungsstücks (14'''') über einen Aufsatz (302) verlängert ist, der mit dem Inneren des Führungsstücks über einen Durchgang (304) für den Durchgang der Nadel (34) verlängert ist, wobei der freie Rand (306) des genannten Aufsatzes gegen den Stopfen (22) des genannten Fläschchens angeordnet ist und zwar so, daß der genannte Aufsatz einen umschlossenen Begrenzungsraum für die Gase bildet, die aus dem genannten Fläschchen austreten können.

53. Spritzenvorrichtung gemäß Anspruch 9,
dadurch **gekennzeichnet**, daß
sie ausserdem einen Nadelschutz (180) enthält, der verschiebbar um die genannte Nadel angebracht ist, wobei der genannte Nadelschutz ein perforierendes Ende umfaßt, das sich zu und von der Spritze der genannten Nadel erstreckr, wobei das genannte perforierende Ende geeignet ist, den Stopfen des genannten Fläschchens zu perforieren.

54. Spritzenvorrichtung gemäß Anspruch 53,
dadurch **gekennzeichnet**, daß
der genannte Nadelschutz (180) an seinem Fixierende (186) einen Teilbereich (187) aus elastischem Material umfaßt, der dicht mit dem Ende der genannten Spritze (12) verbunden ist, auf die die Nadel (34) montiert ist.

55. Spritzenvorrichtung gemaß einem der Ansprüche 1 bis 54,
dadurch **gekennzeichnet**, daß
sie ausserdem einen Bedienungsbolzen eines Kolbens der Spritze aufweist, wobei der genannte Bolzen im genannten Kolben (52) verschraubbar ist.

56. Spritzenvorrichtung gmeäß einem der Ansprüche 1 bis 55,
dadurch **gekennzeichnet**, daß
sie ausserdem eine Selbst-Injektionsvorrichtung umfaßt, die auf dem Zusammenbau aus der genannten Spritze (12) und den genannten Führungselementen (14, 14', 14'', 14''') nach Ablösung des genannten Fläschchens (10) montierbar ist, wobei die genannte Selbst-Injektionsvorrichtung eine Unterlage (202) der Führungselemente auf der genannten Unterlage, erste Drückerelemente (212) zum Hineindrücken des Spritzenkörpers (12) in die genannten Führungselemente (14''') in einer ersten vorbestimmten Entfernung und zweite Drückerelemente (216) zum Hineindrücken des genannten Kolbens (52) in den Körper der genannten Spritze in einer zweiten vorbestimmten Entfernung umfaßt, ohne die Relativstellung der Spritze und der Führungselemente zu verändern, wobei das genannte erste Ende der Führungselemente eine Druckzone auf dem Körper des Patienten bildet.

57. Spritzenvorrichtung gemäß einem der Ansprüche 1 bis 56,
dadurch **gekennzeichnet**, daß
sie Steuerelemente für das Herausziehen der Spritze aus den genannten Steuerelementen enthält, welche Elemente, um das Herausziehen der Spritze aus den Führungselementen teilweise nur so zuzulassen, daß die Verbindungselemente in den genannten Führungselementen verbleiben, und Elemente umfassen, um das vollständige Herausziehen der Spritze nur zuzulassen, nachdem auf die Spritze eine Drehbewegung gegen die Führungselemente ausgeübt worden ist.

58. Spritzenvorrichtung gemäß Anspruch 57
dadurch **gekennzeichnet**, daß
die genannten Steuerelemente einen Sporn (322), der aus der Spritze herausragt, und eine Nutführung (324) umfassen, die nicht parallel zur Richtung des Herausziehens der Spritze verläuft, wobei der genannte Sporn mit der genannten Nutführung verbunden ist, wenn sich die genannte Spritze in den genannten Führungselementen befindet.

59. Spritzenvorrichtung gemaß Anspruch 58,
dadurch **gekennzeichnet**, daß
die genannte Nutführung an der Innenseite der genannten ersten Dichtungselemente vorgesehen ist.
